(19) Europäisches Patentamt
European Patent Office
Office européen des brevets

(11) Numéro de publication: **0 209 435 B1**

(12) # FASCICULE DE BREVET EUROPEEN

(45) Date de publication de fascicule du brevet: **24.11.93**

(51) Int. Cl.$^5$: **C07D 209/08**, C07D 209/34, A61K 31/40, C07D 405/12

(21) Numéro de dépôt: **86401408.9**

(22) Date de dépôt: **26.06.86**

Le dossier contient des informations techniques présentées postérieurement au dépôt de la demande et ne figurant pas dans le présent fascicule.

(54) **Dérivés de l'hydroxy alkoxy 4-phénylpropyl indole, leurs sels, procédé et intermédiaires de préparation, application à titre de médicaments et compositions les renfermant.**

(30) Priorité: **27.06.85 FR 8509785**

(43) Date de publication de la demande:
**21.01.87 Bulletin 87/04**

(45) Mention de la délivrance du brevet:
**24.11.93 Bulletin 93/47**

(84) Etats contractants désignés:
**AT BE CH DE FR GB IT LI LU NL SE**

(56) Documents cités:
**EP-A- 0 099 766**
**DE-A- 3 343 671**
**US-A- 4 387 100**

(73) Titulaire: **ROUSSEL-UCLAF**
**35, Boulevard des Invalides**
**F-75007 Paris(FR)**

(72) Inventeur: **Clemence, François**
**2, rue Turgot**
**F-75009 - Paris(FR)**
Inventeur: **Guillaume, Jacques**
**62, rue Pexerecourt**
**F-75020 - Paris(FR)**
Inventeur: **Hamon, Gilles**
**40, rue de Bagneux**
**F-92120 - Montrouge(FR)**

(74) Mandataire: **Vieillefosse, Jean-Claude et al**
**Département des Brevets**
**ROUSSEL UCLAF**
**B.P no 9**
**F-93230 Romainville (FR)**

EP 0 209 435 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux dérivés de l'hydroxy alkoxy-4-phénylpropyl indole ainsi que leurs sels, le procédé et des intermédiaires de préparation, l'application à titre de médicaments de ces nouveaux dérivés et les compositions les renfermant.

Dans le brevet européen EP 0.099.766 sont décrits des produits dérivés de la 1,3-dihydro 4-(1-hydroxy 2-aminoéthyl) 2H-indol-2-one. Ces produits présentent cependant des propriétés pharmacologiques différentes de celles des produits de la présente demande.

L'invention a pour objet de nouveaux dérivés de l'hydroxy alkoxy 4-phénylpropyl indole, ainsi que leurs sels d'addition avec les acides minéraux ou organiques, caractérisés en ce qu'ils répondent à la formule générale (I) :

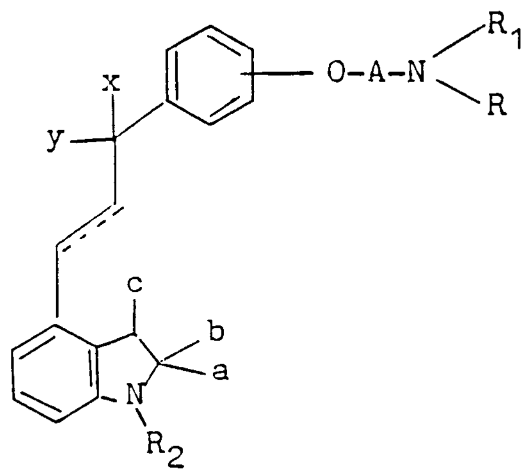

I

dans laquelle R et $R_1$ représentent, indépendamment l'un de l'autre, un atome d'hydrogène, un radical alkyle linéaire renfermant de 1 à 5 atomes de carbone ou un radical alkyle ramifié renfermant de 3 à 5 atomes de carbone, a représente ensemble avec b une fonction oxo, ou représente ensemble avec c une liaison carbone-carbone, b représente un atome d'hydrogène, ou ensemble avec a une fonction oxo, c représente un atome d'hydrogène, ou ensemble avec a représente une liaison carbone-carbone, le pointillé représente la présence éventuelle d'une liaison carbone-carbone, A représente une chaîne

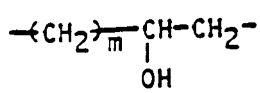

dans laquelle m a la valeur 1, $R_2$ représente un atome d'hydrogène, un radical alkyle linéaire renfermant de 1 à 5 atomes de carbone ou un radical alkyle ramifié renfermant de 3 à 5 atomes de carbone, x représente un radical hydroxyle, ou un atome d'hydrogène, ou ensemble avec y, une fonction oxo et y représente un atome d'hydrogène ou, ensemble avec x, une fonction oxo.

Dans la formule générale (I) et dans ce qui suit, le terme radical alkyle linéaire renfermant de 1 à 5 atomes de carbone désigne, de préférence, un radical méthyle, éthyle ou propyle ; le terme radical alkyle ramifié renfermant de 3 à 5 atomes de carbone désigne, de préférence, un radical isopropyle ou tert-butyle, Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléique, fumarique, succinique, tartrique, citrique, oxalique, glyoxylique, aspartique, alcane sulfoniques tels que les acides méthane et éthane sulfoniques, arylsulfoniques, tels que les acides benzène et paratoluène sulfoniques et arylcarboxyliques.

Parmi les produits objet de l'invention, on peut citer notamment le dérivés répondant à la formule (I) ci-dessus, ainsi que leurs sels d'addition avec les acides minéraux ou organiques, caractérisés en ce que, dans ladite formule (I), a et c représentent ensemble une liaison carbone-carbone.

Parmi ceux-ci, on peut citer plus particulièrement les dérivés caractérisés en ce que dans ladite formule (I), $R_1$ et $R_2$ représentent un atome d'hydrogène et tout particulièrement la 1-/2-/3-/(1,1-diméthyléthyl) amino/ 2-hydroxypropoxy/ phényl/ 3-(1H-indol-4-yl) 1-propanone ainsi que ses sels d'addition avec les

acides minéraux ou organiques.

Parmi les produits objet de l'invention, on peut encore citer notamment les dérivés répondant à la formule (I) ci-dessus, ainsi que leurs sels d'addition avec les acides minéraux ou organiques, caractérisés en ce que, dans ladite formule (I), a et b représentent ensemble une fonction oxo.

Parmi ceux-ci, on peut citer plus particulièrement les dérivés caractérisés en ce que dans ladite formule (I), $R_1$ représente un radical propyle et $R_2$ représente un atome d'hydrogène et tout particulièrement la 1,3-dihydro 4-/3-/2-/3-propylamine 2-hydroxypropoxy/ phényl/ propyl/ 2H-indol-2-one, ainsi que ses sels d'addition avec les acides minéraux ou organiques.

L'invention a également pour objet un procédé de préparation des dérivés tels que définis par la formule (I) ci-dessus, ainsi que de leurs sels, caractérisé en ce que l'on fait réagir un dérivé de formule (II) :

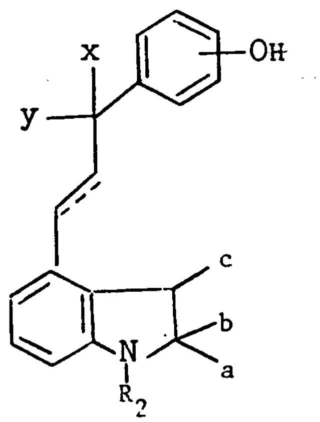

(II)

dans laquelle $R_2$, a, b, c, x, y et le pointillé ont la signification déjà indiquée, avec un halogénure de formule (III) :

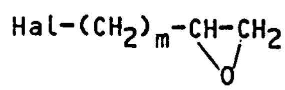

(III)

dans laquelle m a la signification déjà indiquée et Hal représente un atome de chlore, de brome ou d'iode, pour obtenir un produit de formule (IV) :

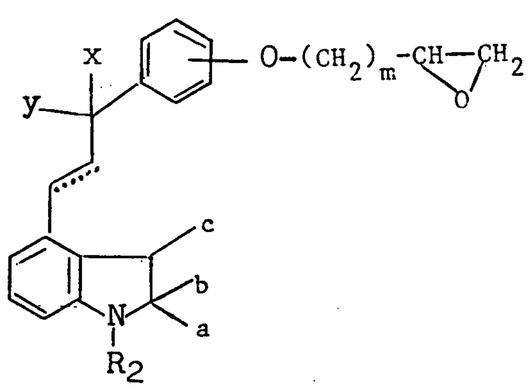

(IV)

dans laquelle $R_2$, a, b, c, x, y, m et le pointeillé ont la signification déjà indiquée, que l'on fait réagir avec une amine de formule (V) :

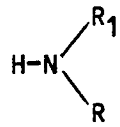

(V)

dans laquelle R et $R_1$ ont la signification déjà indiquée, pour obtenir un produit de formule (I), que l'on isole et, désiré, salifie.

La réaction du dérivé de formule (II) avec l'halogénure de formule (III) est réalisée de préférence en présence d'une base telle que le carbonate de potassium ou de sodium, la soude, la potasse. Dans l'halogénure de formule (III), Hal représente de préférence un atome de chlore.

La réaction du dérivé de formule (IV) avec l'amine de formule (V) est réalisée soit directement en utilisant l'amine comme solvant, soit en utilisant un solvant tel qu'un alcool aliphatique comme le méthanol ou l'éthanol.

L'invention a aussi pour objet un procédé selon ce qui précède, caractérisé en ce que l'on fait réagir un produit de formule ($II_A$) :

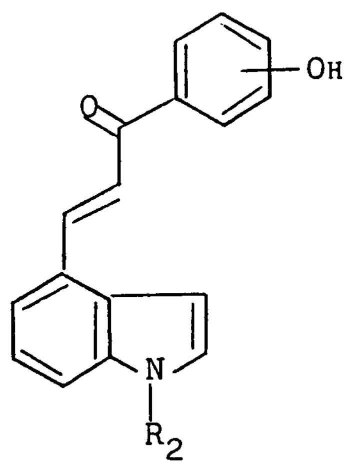

($II_A$)

dans laquelle $R_2$ a la signification déjà indiquée, avec un halogénure de formule (III) :

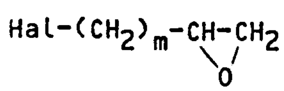

(III)

dans laquelle Hal et m ont la signification déjà indiquée, pour obtenir un produit de formule ($IV_A$) :

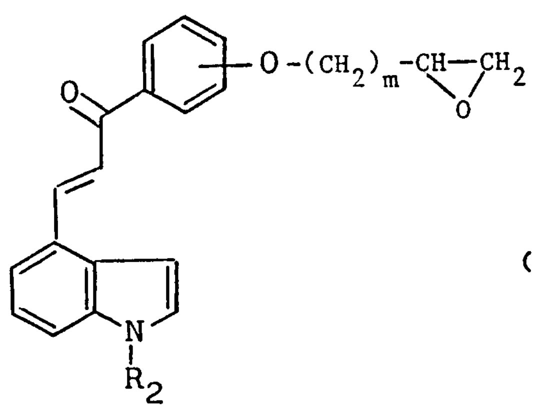

($IV_A$)

dans laquelle $R_2$ et m ont la signification déjà indiquée, que l'on fait réagir avec une amine de formule (V) :

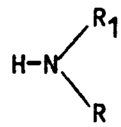

(V)

dans laquelle R et $R_1$ ont la signification déjà indiquée, pour obtenir un produit de formule ($I_A$) :

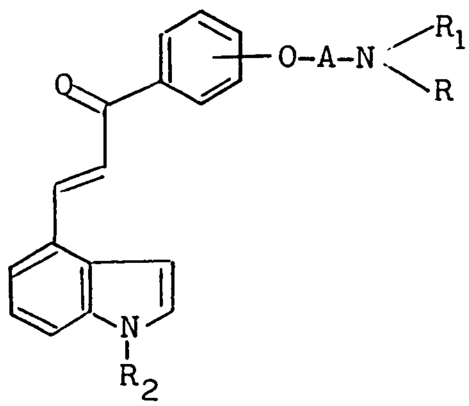

$I_A$

dans laquelle R, $R_1$, et $R_2$ ont la signification déjà indiquée, que soit l'on réduit au niveau de la double liaison de la chaîne aliphatique, pour obtenir un produit de formule $I_B$ :

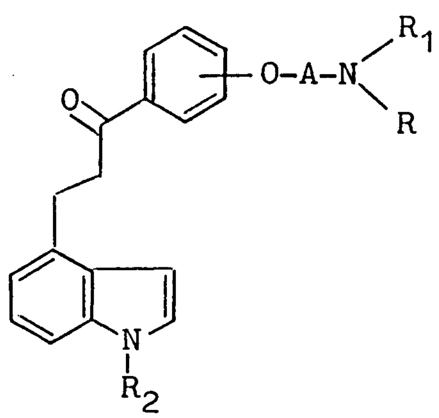

$I_B$

dans laquelle A, R, $R_1$ et $R_2$ ont la signification déjà indiquée, que ou bien l'on isole et, si désiré, salifie, ou bien l'on réduit au niveau de la fonction cétone pour obtenir un produit de formule $I_C$ :

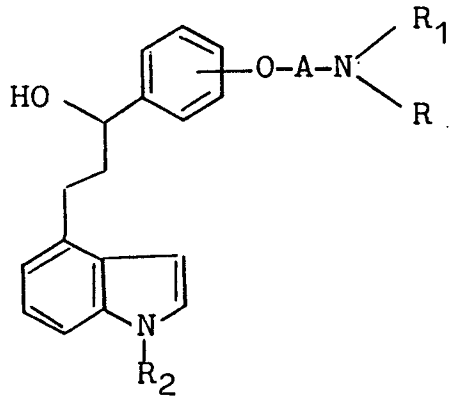

$I_C$

dans laquelle A, R, $R_1$ et $R_2$ ont la signification déjà indiquée, que ou bien l'on isole et, si désiré, salifie, ou bien l'on réduit au niveau de la fonction hydroxy, pour obtenir un produit de formule $I_D$ :

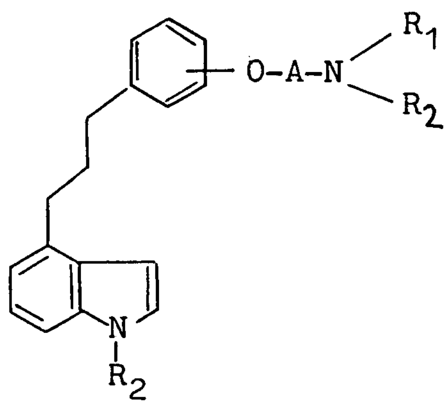

$I_D$

dans laquelle A, R, $R_1$ et $R_2$ ont la signification déjà indiquée, que l'on isole et si désiré salifie, soit l'on réduit ledit produit de formule $I_A$ au niveau de la fonction cétone pour obtenir un produit de formule $I_E$ :

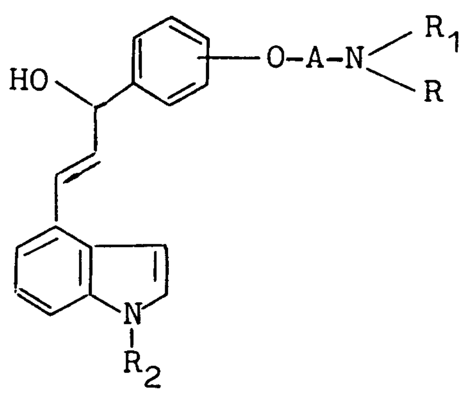

$I_E$

dans laquelle A, R, $R_1$ et $R_2$ ont la signification déjà indiquée,
que ou bien l'on isole, ou bien l'on réduit au niveau de la double liaison de la chaîne aliphatique, pour obtenir un produit de formule $I_D$ que l'on isole et, si désiré, salifie,
soit l'on réduit ledit produit de formule $I_A$ à la fois au niveau de la fonction cétone et celui de la double liaison, pour obtenir un produit de formule $I_D$ que l'on isole et, si désiré salifie,
puis soumet si désiré lesdits produits de formule $I_A$, $I_B$, $I_C$, $I_D$
et $I_E$ à la'action d'un agent d'halogénation pour obtenir un produit de formule (VI) :

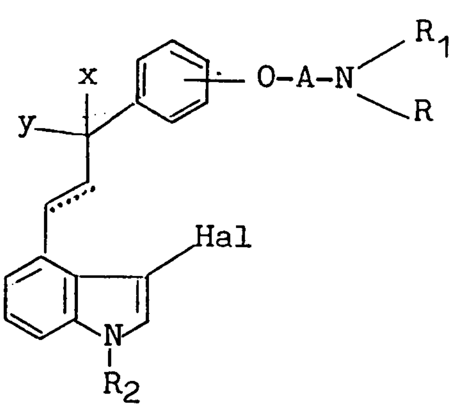

VI

dans laquelle Hal représente un atome de brome ou de chlore, et x, y, A, R, $R_1$, $R_2$ et le pointillé ont la signification déjà indiquée, que l'on soumet à une hydrolyse pour obtenir un produit de formule $I_F$ :

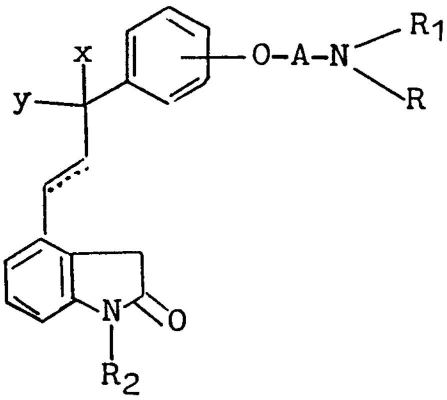

$I_F$

dans laquelle x, y, A, R, $R_1$, $R_2$ et le pointillé ont la signification déjà indiquée, que l'on isole et, si désiré, salifie.

La réduction de la double liaison du produit de formule ($I_A$) est effectuée par de l'hydrogène gazeux en présence d'un catalyseur à base de platine ou de palladium, dans un solvant tel qu'un alcanol renfermant de 1 à 5 atomes de carbone, ou par de l'hydrogène gazeux en présence de Nickel de Raney dans un solvant tel que l'acétate d'éthyle, ou encore par action du sodium dans l'ammoniac dans un solvant tel que le tétrahydrofuranne, le temps du contact étant de moins de 3 heures et, de préférence, d'environ 1 heure.

La réduction de la fonction cétone du produit de formule ($I_B$) est effectuée par un borohydrure ou un cyanoborohydrure alcalin, par exemple de sodium ou de potassium et, de préférence, par la borohydrure de sodium.

La réduction de la fonction hydroxy du produit de formule ($I_C$) est effectuée de préférence par action du sodium dans l'ammoniac, le temps de contact étant d'environ 6 heures. On opère de préférence en présence d'un solvant tel que le tétrahydrofuranne.

La réduction de la fonction cétone du produit de formule ($I_A$) est effectuée par le complexe formé entre un borohydrure alcalin, de préférence le borohydrure de sodium, et la pyridine. La réaction est effectuée de préférence dans un alcanol renfermant au plus 5 atomes de carbone, tel que l'éthanol.

La réduction de la double liaison du produit de formule ($I_E$) est effectuée de préférence par le sodium dans l'ammoniac.

La réduction simultanée de la fonction cétone et de la double liaison du produit de formule ($I_A$) est effectuée de préférence par le sodium dans l'ammoniac.

L'halogénation des produits de formules ($I_A$), ($I_B$), ($I_C$) ($I_D$) et ($I_E$) peut être réalisée, par exemple, à l'aide du complexe bromé de la pyridine de formule :

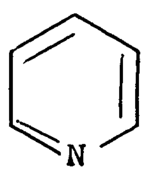

, $Br_2$ , HBr

dans le cas de la bromation. Elle est réalisée avantageusement à l'aide d'un N-halo succinimide, de préférence le N-bromo ou le N-chloro succinimide : on opère dans le dioxanne ou de préférence dans l'acide acétique. Le produit de formule (VI) obtenu est de préférence un produit chloré.

Lorsque l'un des substituants R ou $R_1$ représente un atome d'hydrogène, il peut être utile d'effectuer la réaction d'halogènation sur un produit de formule (I) dont la fonction amine secondaire est bloquée. Le blocage peut être effectué par les moyens classiques connus de l'homme de métier. Il est effectué de préférence par un radical trifluoroacétyle, en opérant à l'aide d'anhydride trifluoroacétique en présence d'une base telle que la triéthylamine, dans un solvant tel que le chloroforme.

L'hydrolyse du produit de formule (VI) est réalisée à l'aide d'un acide minéral tel que l'acide phosphorique, l'acide sulfurique, ou de préférence l'acide chlorhydrique en solution aqueuse concentrée ou,

de préférence, diluée par exemple en solution normale. On peur utiliser, en outre, un solvant tel qu'un alcool aliphatique comme l'éthanol.

Les produits de formule II peuvent être préparés comme suit : On fait réagir un dérivé de formule (VII) :

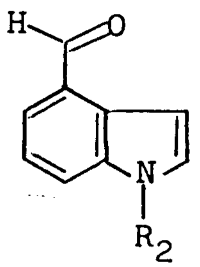

VII

dans laquelle $R_2$ a la signification déjà indiquée, avec un dérivé de formule (VIII) :

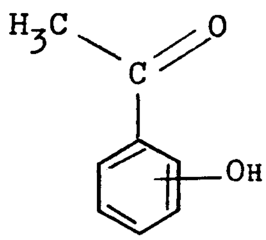

VIII

pour obtenir un produit de formule $II_A$ :

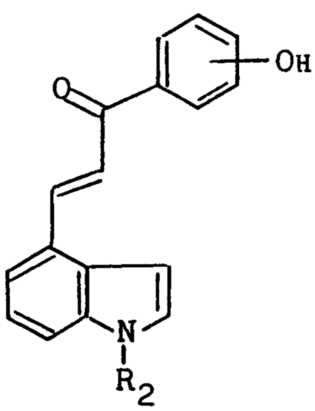

$II_A$

dans laquelle $R_2$ a la signification déjà indiquée, que

soit l'on réduit au niveau de la double liaison de la chaîne aliphatique, pour obtenir un produit de formule $II_B$ :

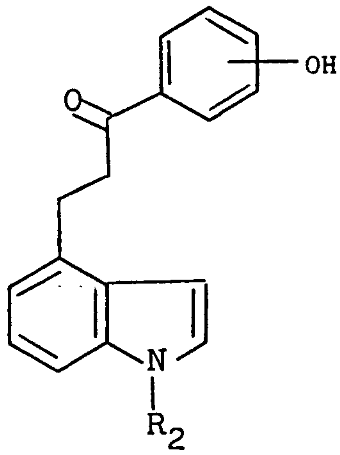

$II_B$

dans laquelle $R_2$ a la signification déjà indiquée, que ou bien l'on isole si désiré, ou bien l'on réduit au niveau de la fonction cétone pour obtenir un produit de formule $II_C$ :

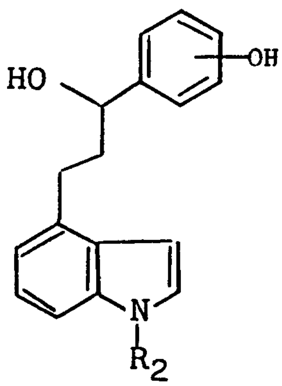

$II_C$

dans laquelle $R_2$ a la signification déjà indiquée, que soit l'on isole si désiré, soit l'on réduit au niveau de la fonction hydroxy, pour obtenir un produit de formule $II_D$ :

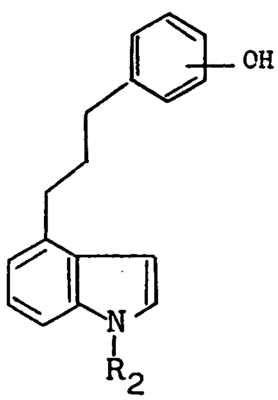

$II_D$

dans laquelle $R_2$ a la signification déjà indiquée, que l'on isole si désiré,

ou bien l'on réduit le produit de formule ($II_B$) au niveau de la fonction cétone, pour obtenir directement un produit de formule ($II_D$), dans laquelle $R_2$ a la signification déjà indiquée,

soit l'on réduit ledit produit de formule $II_A$ au niveau de la fonction cétone, pour obtenir un produit de formule $II_E$ :

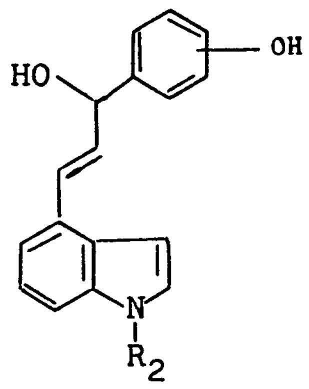

$II_E$

dans laquelle $R_2$ a la signification déjà indiquée, que ou bien l'on isole, ou bien l'on réduit au niveau de la double liaison de la chaîne aliphatique, pour obtenir un produit de formule $II_D$ que l'on isole si désiré,
soit l'on réduit ledit produit de formule $II_A$ à la fois au niveau de la fonction cétone et à celui de la double liaison, pour obtenir un produit de formule $II_D$ que l'on isole si désiré,
puis soumet si désiré lesdits produits de formule $II_A$, $II_B$, $II_C$, $II_D$ et $II_E$ à l'action d'un d'halogénation pour obtenir un produit de formule IX :

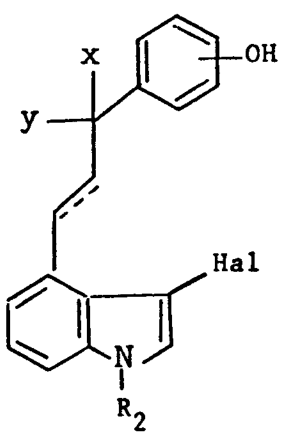

IX

dans laquelle Hal représente un atome de brome ou de chlore, et x, y, $R_2$ et le pointillé ont la signification déjà indiquée, que l'on soumet à une hydrolyse pour obtenir un produit de formule $II_F$:

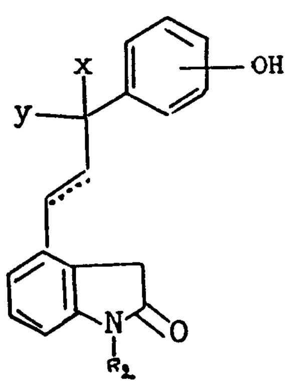

$II_F$

dans laquelle x, y, $R_2$ et le pointillé ont la signification déjà indiquée, que l'on isole si désiré.

La réaction du 4-formyl indole de formule VII avec le dérivé de formule VIII est réalisée de préférence en présence d'une base minérale telle que l'hydroxde de sodium ou de potassium, par exemple au sein d'un solvant tel qu'un alcanol renfermant de 1 à 5 atomes de carbone, notamment l'éthanol.

Les conditions dans lesquelles des différentes réductiond des produits de formule (II) sont effectuées, sont identiques à celles qui ont été décrites précédemment pour les produits de formule (I).

La réduction conduisant directement du product ($II_B$) au produit ($II_D$) est effectuée de préférence à l'aide d'hydrazine en présence de lessive de potasse, dans l'éthylène glycol.

Les conditions dan lesquelles sont effectuées l'halogénation des produits de formule (II) et l'hydrolyse du produit de formule (IX) sont identiques à celles que ont été décrites précédemment pour les produits de formule (I).

Les produits de formule VII sont connus, ou peuvent être préparés comme indiqué, notamment dans J. Org. Chem. 1980 45 p.3350 et suivantes.

Les dérivés de formule (I) présentent un caractère basique. On peut avantageusement préparer les sels d'addition des dérivés de formule (I) en faisant réagir, en proportions sensiblement stoechimétriques, un acide minéral ou organique avec ledit dérivé de formule (I). Les sels peuvent être préparés sans isoler les bases correspondantes.

Les dérivés objet de la présente invention, possèdent de très intéressantes propriétés pharmacologiques ; ils sont doués notamment de remarquables propriétés antiarythmiques et bloquantes des canaux calcicosodiques lents.

Ces propriétés sont illustrées plus loin dans la partie expérimentale.

Ces propriétés justifient l'utilisation des dérivés de l'hydroxy alkoxy 4-phénylpropyl indole de formule (I), ainsi que de leurs sels, à titre de médicaments.

La présente demande a ainsi églament pour objet l'application, à titre de médicaments, des dérivés de l'hydroxy alkoxy 4-phénylpropyl indole, tels que définis par la formule (I), ainsi que de leurs sels d'addition avec les acides pharmaceutiquement acceptables.

Parmi les médicaments, objet de l'invention, on retient, de préférence, les médicaments caractérisés en ce qu'ils sont constitués par les nouveaux dérivés de l'hydroxy alkoxy 4-phénylpropyl indole répondant à la formule (I) dans laquelle a et c représentent ensemble une liaison carbone-carbone ainsi que par leurs sels d'addition avec les acides pharmaceutiquement acceptables.

Parmi ceux-ci, on retient notamment ceux répondant à la formule (I), dans laquelle $R_1$ et $R_2$ représentent un atome d'hydrogène ainsi que leurs sels d'addition avec les acides pharmaceutiquement acceptables.

Parmi ces derniers, on retient tout particulièrement le dérivé dont le nom suit la 1-/2-/3-/(1,1-diméthyléthyl) amino/ 2-hydroxypropoxy/ phényl/ 3-(1H-indol-4-yl) 1-propanone ainsi que ses d'addition avec les acides pharmaceutiquement acceptables.

Parmi les médicaments, objet de l'invention, on retient également, de préférence, les médicaments caractérisés en ce qu'ils sont constitués par les nouveaux dérivés de l'hydroxy alkoxy 4-phénylpropyl indole répondant à la formule (I) dans laquelle a et b représentent ensemble une fonction oxo, ainsi que par leurs sels d'addition avec les acides pharmaceutiquement acceptables.

Parmi ceux-ci, on retient notamment ceux répondant à la forumle (I), dans laquelle $R_1$ représente un radical propyle et $R_2$ représente un atome d'hydrogène, ainsi que leurs sels d'addition avec les acides pharmaceutiquement acceptables.

Parmi ces derniers, on retient tout particulièrement le dérivé dont le nom suit : la 1,3-dihydro 4-/3-/2-/3-propylamino 2-hydroxypropoxy/ phényl/ propyl/ 2H-indol-2-one, ainsi que ses sels d'addition avec les acides pharmaceutiquement acceptables.

Les médicaments selon l'invention trouvent leur emploi, par exemple dans le traitement de l'insuffisance cardiaque, de l'angor sous toutes ses formes, et dans le traitment des arythmies.

La dose usuelle, variable selon le dérivé utilisé, le sujet et l'affection en cause peut être par exemple, de 50 mg à 1 g par jour. Par voie orale, chez l'homme, le dérivé de l'exemple pour le traitement des arythmies ventriculaires, supraventriculaires et jonctionnelles, soit environ de 1,5 mg à 6 mg par kilogramme de poids corporel.

L'invention a également pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés, simples ou dragéifiés, les gélules, les granulés, les suppositoires, les préparations injectables ; ellessont préparées selon les méthodes usuelles. Le ou les principes actifs peuvent y être incoporés à des

excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention s'étend en outre aux produits industriels nouveaux répondant à la formule (IV) :

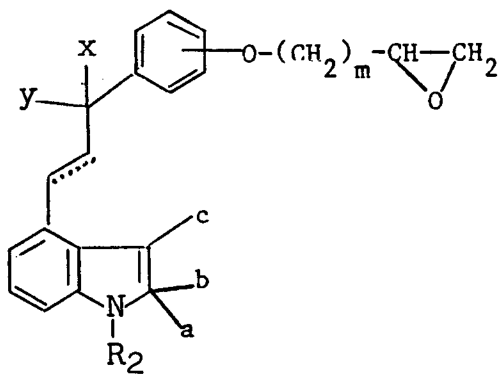

IV

dans laquelle $R_2$, a, b, c, x, y, m et le pointillé ont la signification déjà indiquée, ainsi qu'à ceux répondant à la formule (VI) :

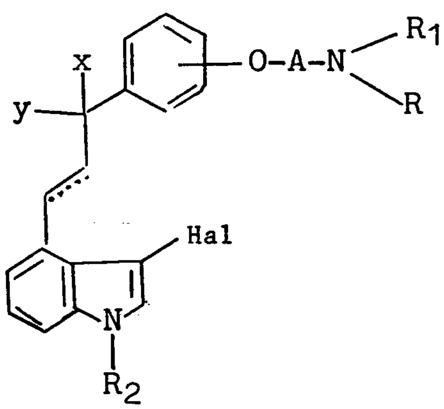

VI

dans laquelle R, $R_1$, $R_2$, x, y, A, Hal et le pointillé ont la signification déjà indiquée.

Les exemples qui suivent illustrent la présente invention sans toutefois la limiter.

**Exemple 1 : 1-/2-/3-/(1,1-diméthyléthyl) amino/ 2-hydroxypropoxy/ phényl 3-1H-indol-4-yl/ 1-propanone et son (E) butène dioate (1,2).**

**Stade A** : 1-/2-(2-oxiranyl) méthoxy/ phényl/ 3-(1H-indol-4-yl) 1-propanone.

On ajoute 9,54 g de carbonate de potassium, puis 16,2 cm3 d'épichlorhydrine à une solution de 4,58 g de 1-/2-hydroxyphényl/ 3-/1H-indol-4-yl/ 1-propanone dans 80 cm3 d'acétone, porte au reflux pendant 43 heures sous atmosphère inerte, filtre, évapore le solvant, purifie par chromatographie sur silice (éluant : dichloroéthane-acétate d'éthyle 95-5) et obtient 5,00 g du produit attendu. F = 72¤C.

**Spectre UV** (éthanol)

| | | |
|---|---|---|
| max. 214 nm | $E^1_1$ = 1625 | $\epsilon$ = 52200 |
| max. 248 nm | $E^1_1$ = 307 | $\epsilon$ = 9900 |
| max. 270 nm | $E^1_1$ = 269 | $\epsilon$ = 8650 |
| max. 278 nm | $E^1_1$ = 268 | |
| max 288 nm | $E^1_1$ = 232 | $\epsilon$ = 7450 |
| infl 300 nm | $E^1_1$ = 118 | $\epsilon$ = 3800 |

Stade B : 1-/2-/3-/(1,1-diméthyléthyl) amino/ 2-hydroxypropoxy/ phényl/ 3-1H-indol-4-yl/ 1-propanone et son (E) butène dioate (1,2).

On porte au reflux pendant 2 heures 30 minutes une solution de 1,25 g de produit du stade A et de 4,1 cm3 de tert-butylamine dans 15 cm3 de méthanol, évapore à sec, purifie par chromatographie sur silice (éluant : chloroforme-méthanol-triéthylamine 8-1-1) et obtient 1,52 g du produit attendu.

**Formation du (E) butène dioate (1,2).**

On ajoute 0,49 g d'acide fumarique dissous à chaud dans 25 cm3 d'éthanol a 80¤C à une solution de 3,36 g de base ci-dessus dans 55 cm3 d'éthanol, filtre, sèche sous pression réduite le précipité obtenu et recueille 2,46 g de produit attendu après recristallisation dans le méthanol. F = 209¤C.

| Analyse : $C_{24}H_{30}N_2O_3$, 1/2 $C_4H_4O_4$ = 452,555 | | | | | | |
|---|---|---|---|---|---|---|
| Calculé<br>Trouvé | C% | 69,0<br>68,8 | H% | 7,13<br>7,2 | N% | 6,14<br>6,3 |

**Préparation de la 1-/2-hydroxyphényl/ 3-/1H-indole-4-yl/ 1-propanone.**

Stade 1 : 1-(2-hydroxyphényl) 3-(1H-indol-4-yl) 2-propen-1-one.

On ajoute à 30¤C sous agitation et atmosphère inerte 0,5 cm3 de potasse à 38% à un mélange de 0,132 g d'indole 4-carboxaldéhyde, de 0,1 cm3 de 2-hydroxyacétophénone et de 0,189 g de chlorure de triéthylbenzylammonium dans 2 cm3 d'éthanol. Après 23 heures d'agitation à 30¤C, on dilue à l'eau, extrait à l'acétate d'éthyle, lave à l'eau, sèche, amène à sec, purifie par chromatographie sur silice (éluant : chlorure de méthylène) et obtient 159 mg de produit attendu. F ≃ 164¤C.

Stade 2: 1-/2-hydroxyphényl/ 3-/1H-indole-4-yl/ 1-propanone.

On hydrogène à température ambiante jusqu'à fin d'absorption 200 mg de produit du stade 1 dans 10 cm3 de méthanol en présence de palladium sur charbon actif, filtre, évapore le méthanol, purifie par chomatographie sur silice (éluant : cyclohexane-dichlorométhane-triéthylamine 6-3-1) et obtient 143 mg de produit attendu. F ≃ 143¤C.

**Spectre UV** (éthanol)

| | | |
|---|---|---|
| max. 214 nm | $E^1_1$ = 2080 | $\epsilon$ = 55200 |
| max. 254 nm | $E^1_1$ = 555 | $\epsilon$ = 14700 |
| infl 275 nm | $E^1_1$ = 295 | |
| infl 286 nm | $E^1_1$ = 220 | |
| max. 323 nm | $E^1_1$ = 117 | $\epsilon$ = 4700 |

**Exemple 2** : **3-(1H-indol-4-yl) 1-/2-/2-hydroxy 3-(propylamino) propoxy/ phényl/ 1-propanone et son (E) butène dioate (1,2).**

On porte au reflux pendant 45 minutes une solution de 2,85 g de produit du Stade A de l'exemple 1 et 5,12 cm3 de propylamine dans 30 cm3 de méthanol, évapore le solvant, purifie par chromatographie sur silice (éluant : acétate d'éthyle-triéthylamine 9-1) et obtient 3,4 g de produit attendu.

**Formation du (E) butène dioate (1,2).**

On ajoute 1,04 g d'acide fumarique à une solution de 3,4 g de base ci-dessus dans 50 cm3 d'éthanol, filtre et sèche sous pression réduite le précipité obtenu et recueille 1,77 g de produit attendu. F ≃ 183¤C après recristallisation dans le mélange méthanol-éthanol.

| Analyse: $(C_{23}H_{28}N_2O_3)_2$, $C_4H_4O_4$ = 993,127 | | | | | | |
|---|---|---|---|---|---|---|
| Calculé<br>Trouvé | C% | 68,47<br>68,3 | H% | 6,89<br>7,1 | N% | 6,39<br>6,5 |

**Exemple 3 : 1-/2-/3-/(1,1-diméthyléthyl) amino/ 2-hydroxypropoxy/ phényl/ 3-(1H-indol-4-yl) 2-propèn-1-one.**

**Stade A** : 1-/2-/(2-oxiranyl) méthoxy/ phényl/ 3-(1H-indol-4-yl) 2-propèn-1-one.

On ajoute 3,46 g de carbonate de potassium, puis 10,5 cm3 d'épichlorhydrine à une solution de 4 g de 1-/2-hydroxyphényl/ 3-/1H-indol-4-yl/ 2-propèn-1-one dans 80 cm3 d'acétone, porte au reflux pendant 50 heures sous atmosphère inerte, filtre, évapore le solvant, purifie par chromatographie sur silice (éluant : dichlorométhane-acétate d'éthyle (95-5) et obtient 2,26 g de produit attendu.

**Spectre UV** (éthanol)

| | | |
|---|---|---|
| max. 213 nm | $E^1_1$ = 947 | ε = 30200 |
| max. 266 nm | $E^1_1$ = 407 | ε = 13000 |
| infl 340 nm | $E^1_1$ = 262 | |
| max. 390 nm | $E^1_1$ = 442 | ε = 14100 |

**Stade B** : 1-/2-/3-/(1,1-diméthyléthyl) amino/ 2-hydroxypropoxy/ phényl/ 3-(1H-indol-4-yl) 2-propèn-1-one.

On porte au reflux pendant 2 heures 30 minutes une solution de 2,13 g du stade A ci-dessus et de 7,1 cm3 tert-butylamine dans 22 cm3 de méthanol, évapore le solvant et obtient 2,6 g du produit attendu après chromatographie sur silice (éluant : chloroforme-méthanol-triéthylamine 8-1-1).

**Spectre UV** (éthanol)

| | | |
|---|---|---|
| max. 215 nm | $E^1_1$ = 433 | ε = 17000 |
| max. 264 nm | $E^1_1$ = 171 | ε = 6700 |
| infl 347 nm | $E^1_1$ = 114 | |
| max. 338 nm | $E^1_1$ = 174 | ε = 6800 |

**Exemple 4 : 1-/2-/3-/(1,1-diméthyléthyl) amino/ 2-hydroxypropoxy/ phényl/ 3-(1H-indol-4-yl) 1-propanone.**

On hydrogène à température ambiante jusqu'à fin d'absorption 2,6 g de produit de l'exemple 3 dans 50 cm3 de méthanol en présence de palladium sur charbon actif, filtre, évapore le méthanol, purifie par chromatographie sur silice (éluant : chloroforme-acétone-triéthylamine (6-3-1) et obtient 1,97 g de produit attendu.

**Spectre UV** (éthanol)

| | | |
|---|---|---|
| max. 214 nm | $E^1_1$ = 1244 | ε = 49100 |
| max. 247 nm | $E^1_1$ = 220 | ε = 8700 |
| max. 269 nm | $E^1_1$ = 196 | ε = 7700 |
| max. 275 nm | $E^1_1$ = 194 | ε = 7650 |
| infl 286 nm | $E^1_1$ = 164 | |
| infl 300 nm | $E^1_1$ = 85 | ε = 3350 |

**Exemple 5 : 1,3-dihydro 4-/3-/2-/3-/(1,1-diméthyléthyl) amino/ 2-hydroxy propoxy/ phényl/ 3-oxopropyl/ 2H-indol-2-one et son fumarate.**

**Stade A** : 3-(3-chloro 1H-indol-4-yl) 1-/2-/3-/(1,1-diméthyléthyl) amino/ 2-hydroxy propoxy/ phényl/ 1-propanone.

On agite 2 heures à température ambiante 8,6 g de 1-/2-/3-/(1,1-diméthyléthyl) amino/ 2-hydroxy/ propoxy/ phényl/ 3-(1H-indol-4-yl) 1 propanone préparé à l'exemple 1 dans 170 cm3 d'acide acétique avec 3 g de N-chloro succinimide. On dilue à l'eau, alcalinise avec de la soude, extrait à l'acétate d'éthyle, lave à l'eau, sèche et évapore le solvant sous pression réduite. Après purification par chromatographie sur silice (éluant : chloroforme-acétone-triéthylamine 6-3-1), on obtient 6,37 g de produit attendu.

**Stade B** : 1,3-dihydro 4-/3-/2-/3-/(1,1-diméthyléthyl) amino/ 2-hydroxy propoxy/ phényl/ 3-oxopropyl/ 2H-indol-2-one et son fumarate neutre.

On aite 17 heures à température ambiante 6,37 g du produit obtenue au stade A dans 192 cm3 d'acide chlorhydrique N et 192 cm3 d'éthanol. On dilue à l'eau le milieu réactionnel, alcalinise avec de la soude, extrait à l'acétate d'éthyle, lave à l'eau sèche et évapore le solvant sous pression réduite. Après chromatographie sur silice (éluant : chloroforme-acétone-triéthylamine 6-3-1), on obtient 2,32 g du produit attendu sous forme de base.

**Formation du fumarate.**

On dissout à chaud 2,12 g de base ci-dessus dans 80 cm3 d'isopropanol et ajoute 599 mg d'acide fumarique. On filtre les cristaux, les sèche sous pression réduite à 120¤C et récupère 1,77 g de produit brut, que l'on purifie par recristallisation dans un mélange d'éthanol et de méthanol (2-1). On recueille 1,21 de fumarate neutre. F = 238¤C.

| Analyse $(C_{24}H_{30}N_2O_4)1/2\ C_4H_4O_4$ : 468,554. | | | | | | |
|---|---|---|---|---|---|---|
| Calculé<br>Trouvé | C% | 66,65<br>66,3 | H% | 6,88<br>6,9 | N% | 5,98<br>5,8. |

**Exemple 6 : 1,3-dihydro 4-/3-oxo 3-/2-/2-hydroxy 3-(propylamino) propoxy/ phényl/ propyl/ 2H-indol-2-one et son oxalate acide.**

**Stade A** : 3-(3-chloro 1H-indol-4-yl) 1-/2-/2-hydroxy 3-(propylamino) propoxy/ phényl/ 1-propanone.

On opère comme à l'exemple 5, stade A, à partir de 6,3 g de la base du produit obtenu à l'exemple 2, 120 cm3 d'acide acétique et 2,43 g de N-chlorosuccinimide. On obtient 5,6 g de produit attendu.

**Stade B** : 1,3-dihydro 4-/3-oxo 3-/2-/2-hydroxy 3-(propylamino) propoxy/ phényl/ propyl/ 2H-indol-2-one et son oxalate acide.

On opère comme à l'exemple 5 stade B à partir de 5,6 g du produit obtenu au stade A. On obtient 2,42 g de produit attendu sous forme de base.

**Formation de l'oxalate acide.**

On dissout à chaud 2,42 g de la base ci-dessus dans 20 cm3 d'éthanol et ajoute 768 mg d'acide oxalique, glace, filtre les cristaux, les sèche sous pression réduit à 80¤C et recueille 1,2 g de produit brut que l'on purifie par recristallisation dans l'éthanol. On obtient 0,9 g de produit attendu. F = 120¤C.

| Analyse $C_{23}H_{28}N_2O_4$), $C_2H_2O_4$ : 486,526. | | | | | | |
|---|---|---|---|---|---|---|
| Calculé<br>Trouvé | C% | 61,72<br>62,0 | H% | 6,21<br>6,1 | N% | 5,76<br>5,8. |

**Exemple 7 : 1-(1,1-diméthyléthyl) amino 3-/2-/3-/1H-indol-4-yl/ propyl/ phénoxy/ 2-propanol et son fumarate neutre.**

**Stade A** : 4-/3-/2-/(2-oxiranyl) méthoxy/ phényl/ propyl/ 1H-indole.

On ajoute à température ambiante sous atmosphère inerte, 0,235 g de carbonate de potassium puis 0,6 cm3 d'épichlorhydrine à une solution de 0,284 g de 2-/3-(1H-indol-4-yl) propyl/ phénol dans 4 cm3 d'acétone. On chauffe au reflux le mélange pendant 8 heures, ajoute de nouveau 1,8 cm3 d'épichlorhydrine et poursuit l'agitation et le chauffage pendant 21 heures. On filtre et élimine le solvant sous pression réduite. Après chromatographie du résidu sur silice (éluant : chlorure de méthylène), on obtient 0,283 g de produit attendu.

**Stade B** : 1-(1,1-diméthyléthyl) amino 3-/2-/3-/1H-indol-4-yl/ propyl/ phénoxy/ 2-propanol et son fumarate neutre.

On chauffe 2 heures à 80¤C, 2,2 g de produit abtenu au stade A et 7,6 cm3 de tert-butylamine dans 20 cm3 de méthanol. On évapore le solvant, chromatographie le résidu sur silice (éluant : chloroforme-acétone-triéthylamine 6-3-1) et obtient 2,7 g de produit attendu sous forme de base. F = 96¤C.

**Formation du fumarate neutre.**

On dissout 1,84 g de la base ci-dessus dans 50 cm3 d'éthanol et ajoute 0,557 g d'acide fumarique à température ambiante sous atmosphère inerte. On filtre les cristaux, les sèche sous pression réduite et obtient 1,67 g de produit brut que l'on purifie par recristallisation dans l'éthanol. On recueille 1,31 g de fumarate neutre. F = 200¤C.

| Analyse $(C_{24}H_{32}N_2O_2)1/2\ C_4H_4O_4$ : 438,57. | | | | | | |
|---|---|---|---|---|---|---|
| Calculé<br>Trouvé | C% | 71,21<br>71,5 | H% | 7,81<br>8,1 | N% | 6,39<br>6,3. |

Le 2-/3-(1H-indol-4-yl) propyl/ phénol utilisé au départ de l'exemple 7 a été préparé comme suit.

Préparation du 2-/3-(1H-indol-4-yl) propyl/ phénol.

On ajoute lentement sous agitation 22,6 cm3 d'hydrate d'hydrazine, puis 11,6 g de 1-/2-hydroxyphényl/ 3-/1H-indol-4-yl/ 1-propanone dont la préparation est donnée après l'exemple 1 à 50 cm3 de diéthylène glycol, ajoute 20 cm3 de lessive de potasse à 38 %, porte à 140¤C pendant 30 minutes sous atmosphère inerte, distille l'eau et l'hydrate d'hydrazine en excès à 210¤C, agite encore pendant 2 heures, refroidit, ajoute de l'eau, extrait à l'acétate d'éthyle, lave à l'eau, sèche, amène à sec le filtrat, purifie le résidu par chromatographie sur silice (éluant : chlorure de méthylène) et obtient 8,613 g du produit attendu. F≃ 89¤C.

Spectre U.V. (éthanol)

| | | |
|---|---|---|
| Max. 219 nm<br>Infl. 260 nm<br>Max.. 273 nm<br>Infl. 278 nm<br>Max. 289 nm<br>Infl. 310 nm<br>Infl. 334 nm. | $E^1_1$ 1 705<br>$E^1_1$ 313<br>$E^1_1$ 380<br>$E^1_1$ 375<br>$E^1_1$ 225 | $\epsilon$ = 42 900<br><br>$\epsilon$ = 9 550<br><br>$\epsilon$ = 5 650 |

**Exemple 8 : 1-/2-/3-(1H-indol-4-yl) propyl/ phénoxy/ 3-propylamino 2-propanol et son fumarate neutre.**

On opère comme stade B de l'exemple 7 à partir de 2,75 g de produit obtenu au stade A de l'exemple 7 et 7,6 cm3 de propylamine. Après chromatographie sur silice (éluant : acétate d'éthyle-triéthylamine 9-1), on obtient 2,62 g de produit attendu sous forme de base. F = 99-100¤C.

**Formation du fumarate neutre.**

On opère comme à l'exemple 7 à partir de 1,8 g de base obtenue ci-dessus et de 0,569 g d'acide fumarique en remplaçant l'éthanol par de l'isopropanol. On obtient 1,26 g de produit attendu. F = 163¤C.

| Analyse $(C_{23}H_{30}N_2O_2)1/2\ C_4H_4O_4$ : 424,55. | | | | | | |
|---|---|---|---|---|---|---|
| Calculé<br>Trouvé | C% | 70,73<br>70,5 | H% | 7,60<br>7,7 | N% | 6,60<br>6,5. |

**Exemple 9 : 1,3-dihydro 4-/3-/3-/2-hydroxy 3-(propylamino) propoxy/ phényl/ propyl/ 2H-indol-2-one.**

**Stade A** : N-/3-/2-/3(1H-indol-4-yl) propyl/ phénoxy/ 2-hydroxy propyl/ N-propyl trifluoroacétamide.

On ajoute à une température inférieure à 10¤C et sous atmosphère inerte, 9,68 cm3 d'anhydride trifluoroacétique dans un mélange comprenant 4,93 g de base obtenue à l'exemple 8, 51 cm3 de chloroforme et 19,3 cm3 de triéthylamine et agite pendant 1 heure. On dilue à l'eau le milieu réactionnel, extrait à l'acétate d'éthyle, lave à l'eau, sèche et évapore le solvant. Après chromatographie du résidu sur silice (éluant : chloroforme-acétone-triéthylamine 6-3-1), on obtient 6,3 g de produit attendu.

**Stade B** : N-/3-/2-/3-(3-chloro 1H-indol-4-yl) propyl/ phénoxy/ 2-hydroxy propyl/ N-propyl trifluoroacétamide.

On mélange sous atmosphère inerte 6,3 g de produit obtenu au stade A, 100 cm3 de dioxanne et 2 g de N-chlorosuccinimide et maintient 19 heures à température ambiante. On dilue à le milieu réactionnel, extrait à l'acétate d'éthyle, lave à l'eau, sèche et évapore le solvant. Après chromatographie du résidu sur silice (éluant : chloroforme-cyclohexane-triéthylamine 6-3-1), on obtient 4,73 g de produit attendu.

Stade C : 1,3-dihydro 4-/3-/2-/2-hydroxy 3-(propylamino) propoxy/ phényl/ propyl/ 2H-indol-2-one.

On chauffe 3 heures au reflux sous atmosphère inerte 4,66 g de produit obtenu au stade B dans 260 cm3 d'acide chlorhydrique N. On dilue à l'eau, alcalinise avec de la soude, extrait à l'acétate d'éthyle, lave à l'eau la phase organique, sèche et évapore le solvant. On empâte le résidu à l'éther éthylique et le sèche sous pression réduite à 100¤C. On obtient 2,19 g de produit attendu que l'on recristallise dans l'isopropanol.
F = 122¤C.

| Analyse $C_{23}H_{30}N_2O_3$ : 382,506. | | | | | | |
|---|---|---|---|---|---|---|
| Calculé<br>Trouvé | C% | 72,22<br>72,1 | H% | 7,90<br>7,9 | N% | 7,32<br>7,3. |

Exemple 10 :

On a préparé des comprimés répondant à la formule :

| | |
|---|---|
| - fumarate neutre de 1-/2-/3-/(1,1-diméthyléthyl) amino/ 2-hydroxypropoxy/ phényl/ 3-/1H-indol-4-yl/ 1-propanone<br>- excipient q. s. pour un comprié terminé à | 100 mg ;<br><br>150 mg. |
| (Détail de l'excipient : lactose, amidon, talc, stéarate de magnésium). | |

Exemple 11 :

On a préparé des comprimés répondant à la formule :

| | |
|---|---|
| - fumarate neutre de 3-(1H-indol-4-yl) 1-/2-/2-hydroxy 3-(propylamino) propoxy/ phényl/ 1-propanone<br>- excipient q.s. pour un comprimé terminé à | 100 mg<br><br>150 mg. |
| (Détail de l'excipient : lactose, amidon, talc, stéarate de magnésium). | |

Etude pharmacologique

1) Action antiarythmique chez le rat

On trachéotomise des rats mâles pesant 300-350 g anesthésiés par voie intrapéritonéale à l'aide de 1,20 g/kg d'uréthane et les soumet à une respiration artificielle (40-50 insufflations de 3 ml/minute).

On implante des aiguilles en sous cutané de manière à enregistrer l'électrocardiogramme des rats sur le signal en dérivation DII.

On administre les produits à tester par voie intraveineuse ou par voie orale.

Cinq minutes après l'administration du produit par voie intraveineuse ou 1 heure après administration par voie orale, on perfuse la veine jugulaire des rats avec 10 µg/mn d'une solution d'aconitine et on note le temps d'apparition des troubles du rythme cardiaque. (10 µg d'aconitine correspondant à la perfusion de 0,2 ml de solution).

Les résultats sont exprimés en pourcentage d'allongement du temps d'apparition des troubles du rythme cardiaque par rapport aux témoins et en fonction de la dose du produit testé.

Les résultats figurant sur le tableau ci-après montrent que les produits de la présente demande sont doués de remarquables propriétés antiarythmiques.

| Produit de l'exemple | voie | Dose en mg/kg | Pourcentage d'allongement du temps |
|---|---|---|---|
| 1 | PO | 25 | 142 |
| | | 10 | 39 |
| | | 5 | 41 |
| | IV | 0,5 | 28 |
| 2 | IV | 1 | 32 |
| 5 | PO | 10 | 47 |
| | IV | 0,5 | 66 |
| 6 | IV | 0,25 | 31 |
| 7 | IV | 2,5 | 41 |
| 8 | IV | 2,5 | 25,5 |
| 9 | IV | 0,5 | 45 |

2) Test d'activité anticalcique in vitro

Des artères caudales de rat découpées en spirale sont reliées à des capteurs de tension et sont maintenues dans des cuves de 25 ml de tampon Krebs-bicarbonate de sodium (NaCl : 120,8 mM, KCl : 5,9 mM, $MgCl_2$ : 1,2 mM, $NaH_2PO_4$ : 1,2 mM, $NaHCO_3$ : 15,5 mM, glucose : 12,6 mM) à 37¤C aérées avec un mélange $O_2$ : 95% - $CO_2$ : 5%.

Les préparations sont dépolarisées par une solution tampon à concentration 100 mM en ions $K^+$ (NaCl : 26,7 mM, KCl : 100 mM, $MgCl_2$ 1,2 mM, $NaH_2PO_4$ : 1,2 mM, $NaHCO_3$ : 15,5 mM, glucose : 12,6 mM).

On ajoute, sous un volume de 250 µl, du chlorure de calcium, de manière à obtenir une gamme de concentrations croissantes en ions $Ca2^+$ allant de 0,1 à 3,0 mM ; on enregistre les contractions des artères et établit ainsi une gamme témoin. On répète l'opération avec la gamme l'ions $Ca^{+2}$ toutes les 15 minutes et la préparation est lavée quatre fois après chaque gamme.

Lorsque l'on obtient une réponse stable, l'on effectue l'opération avec les gammes d'ions $Ca2^+$ en présence de différentes concentrations du produit à tester, jusqu'à ce qu'une réponse stable soit obtenue.

Les contractions des artères dépendent de l'entrée des ions $Ca2^+$ dans les cellules des muscles lisses et sont provoquées par la dépolarisation du muscle lisse par les ions $K^+$ et par l'action de la noradrénaline libérée au niveau présynaptique. En recommençant l'opération avec des artères dénervées par action de 6-OH dopamine, on supprime l'action propre due à la noradrénaline.

Les résultats sont exprimés en CI 50 (concentration inhibitrice 50) concentration du produit testé que inhibe de 50 % la contraction due aux ions $K^+$.

On constate d'après les résultats figurant sur le tableau ci-après que les produits de la présente demande possèdent une bonne activité anticalcique.

| Produit de l'exemple | CI 50 en µM |
|---|---|
| 1 | 0,4 |
| 2 | 3,8 |
| 6 | 3,6 |
| 7 | 0,64 |
| 8 | 1,4 |

3) Etude de la toxicité aigüe

On a évalué les doses létales $DL_0$ des différents composés testés après administration par voie orale chez la souris.

On appelle $DL_0$ la dose maximale ne provoquant aucune mortalité en 8 jours.

Les résultats obtenus sont les suivants :

| Produit de l'exemple | $DL_0$ en mg/kg |
|---|---|
| 1 | 100 |
| 2 | 100 |
| 5 | > 400 |
| 6 | 100 |
| 7 | > 400 |
| 8 | > 400 |

## Revendications

### Revendications pour les Etats contractants suivants : BE, CH, DE, FR, GB, IT, LI, LU, NL, SE

**1.** Les dérivés de l'hydroxy alkoxy 4-phénylpropyl indole ainsi que leurs sels d'additions avec les acides minéraux ou organiques, caractérisés en ce qu'il répondent à la formule générale I :

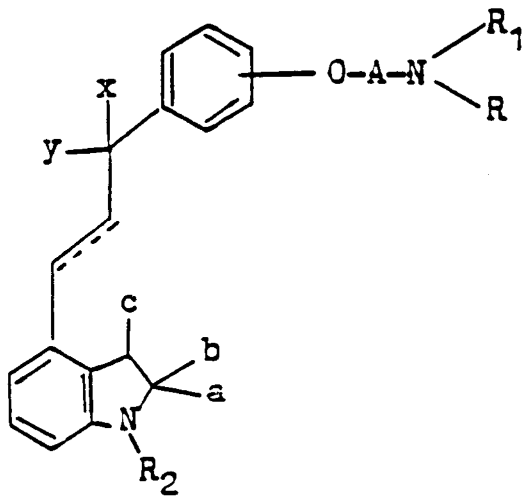

I

dans laquelle R et $R_1$ représentent, indépendamment l'un de l'autre, un atome d'hydrogène, un radical alkyle linéaire renfermant de 1 à 5 atomes de carbone, un radical alkyle ramifié de 3 à 5 atomes de carbone, a représente ensemble avec b une fonction oxo, ou représente ensemble avec c une liaison carbone-carbone, b représente un atome d'hydrogène, ou ensemble avec a une fonction oxo, c représente un atome d'hydrogène, ou ensemble avec a représente une liaison carbone-carbone, le pointillé représente la présence éventuelle d'une liaison carbone-carbone, A représente une chaîne

$$-(CH_2)_m-\underset{\substack{|\\ OH}}{CH}-CH_2-$$

dans laquelle m a a valeur 1, $R_2$ représente un atome d'hydrogène, un radical alkyle linéaire renfermant de 1 à 5 atomes de carbone ou un radical alkyle ramifié renfermant de 3 à 5 atomes de carbone, x représente un radical hydroxyle, ou un atome d'hydrogène, ou ensemble avec y, une fonction oxo et y représente un atome d'hydrogène ou, ensemble avec x, une fonction oxo.

**2.** Les dérivés tels que définis par la formule (I) de la revendication 1, ainsi que leurs sels d'addition avec les acides minéraux ou organiques, caractérisés en ce que a et c représentent ensemble une liaison carbone-carbone.

**3.** Les dérivés selon la revendication 2, ainsi que leurs sels d'addition avec les acides minéraux ou organiques, caractérisés en ce que $R_1$ et $R_2$ représentent un atome d'hydrogène.

**4.** Le dérivé de formule (I) selon la revendication 1 dont le nom suit :
- la 1-/2-/3-/(1,1-diméthyléthyl) amino/ 2-hydroxypropoxy/ phényl/ 3-(1H-indol-4-yl) 1-propanone ainsi que ses sels d'addition avec les acides minéraux ou organiques.

**5.** Les dérivés tels que définis par la formule (I) de la revendication 1, ainsi que leurs sels d'addition avec les acides minéraux ou organiques, caractérisés en ce que a et b représentent ensemble une fonction cétone.

6. Les dérivés selon la revendication 5, ainsi que leurs sels d'addition avec les acides minéraux ou organiques, caractérisés en ce que $R_1$ représente un radical propyle et $R_2$ représente un atome d'hydrogène.

7. Le dérivé de formul (I) selon la revendication 1 dont le nom suit :
   - la 1,3-dihydro 4-/3-/2-/3-propylamino/ 2-hydroxypropoxy/ phényl/ propyl/ 2H-indol-2-one, ainsi que ses sels d'addition avec les acides minéraux ou organiques.

8. Procédé de préparation des dérivés tels que définis par la formule (I) de la revendication 1, ainsi que de leurs sels, caractérisé en ce que l'on fait réagir un dérivé de formule (II) :

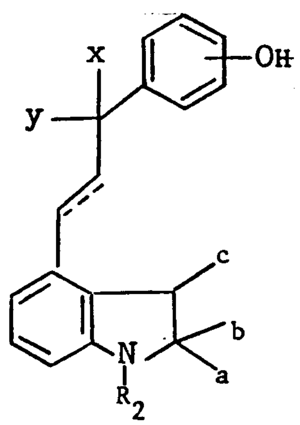

(II)

dans laquelle $R_2$, a, b, c, x, y et le pointillé ont la signification déjà indiquée, avec un halogénure de formule (III) :

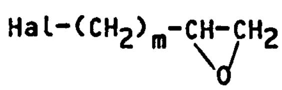

(III)

dans laquelle m a la signification déjà indiquée et Hal représente un atome de chlore, de brome ou d'iode, pour obtenir un produit de formule (IV) :

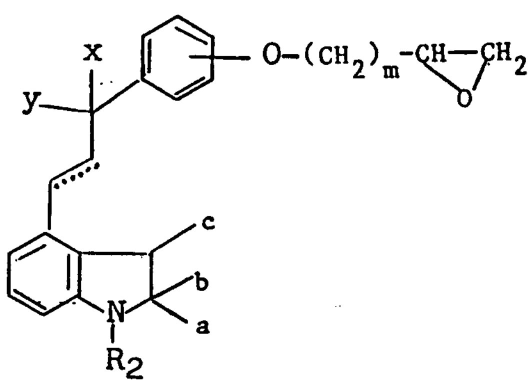

(IV)

dans laquelle $R_2$, a, b, c, x, y, m et le pointillé ont la signification déjà indiquée, que l'on fait réagir avec une amine de formule (V) :

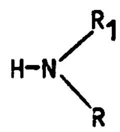

(V)

dans laquelle R et $R_1$ ont la signification déjà indiquée, pour obtenir un produit de formule (I), que l'on isole et, si désiré, salifie.

**9.** Procédé selon la revendication 8, caractérisé en ce que Hal représente un atome de chlore et en ce que la réaction du dérivé de formule (II) avec l'halogénure de formule (III) est utilisée en présence d'une base.

**10.** Procédé selon la revendication 8, caractérisé en ce que l'on fait réagir un produit de formule ($II_A$) :

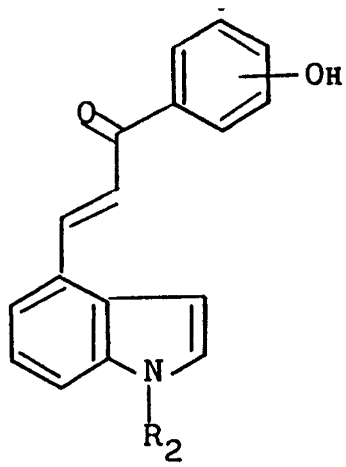

($II_A$)

dans laquelle $R_2$ a la signification déjà indiquée, avec un halogénure de formule (III) :

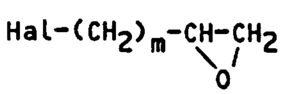

(III)

dans laquelle Hal et m ont la signification déjà indiquée, pour obtenir un produit de formule ($IV_A$) :

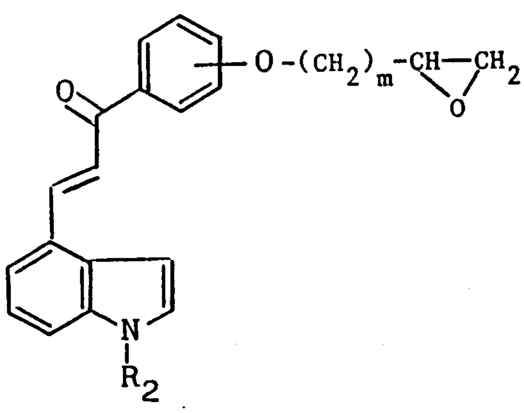

($IV_A$)

dans laquelle $R_2$ et m ont la signification déjà indiquée, que l'on fait réagir avec une amine de formule (V) :

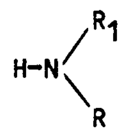

(V)

dans laquelle R et $R_1$ ont la signification déjà indiquée, pour obtenir un produit de formule ($I_A$) :

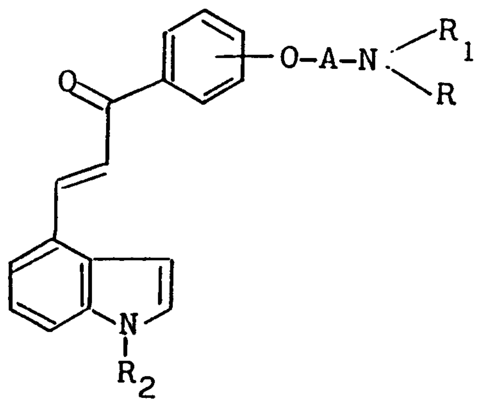

$I_A$

dans laquelle R, $R_1$ et $R_2$ ont la signification déjà indiquée, que
soit l'on réduit au niveau de la double liaison de la chaîne aliphatique, pour obtenir un produit de formule ($I_B$) :

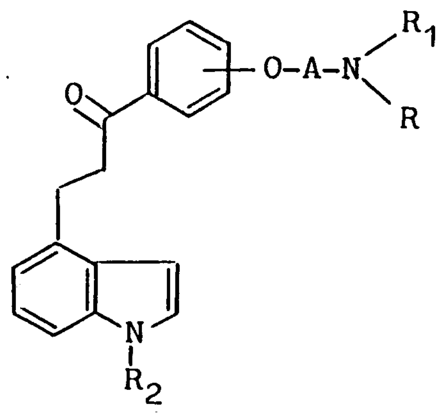

$I_B$

dans laquelle A, R, $R_1$, et $R_2$ ont la signification déjà indiquée, que ou bien l'on isole et, si désiré, salifie, ou bien l'on réduit au niveau de la fonction cétone pour obtenir un produit de formule ($I_C$) :

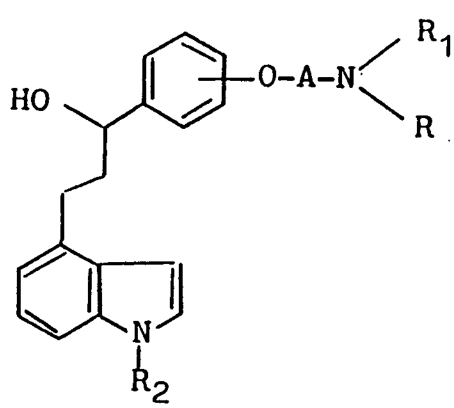

$I_C$

dans laquelle A, R, $R_1$ et $R_2$ ont la signification déjà indiquée, que ou bien l'on et, si désiré, salifie, ou

bien l'on réduit au niveau de la fonction hydroxy, pour obtenir un produit de formule ($I_D$) :

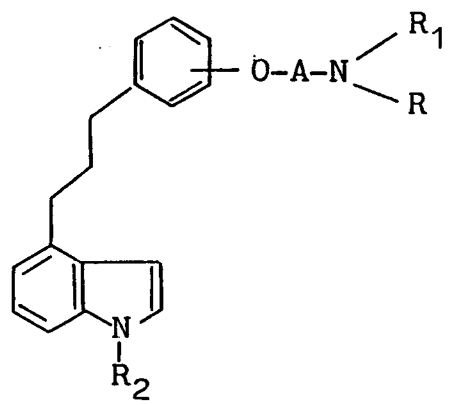

$I_D$

dans laquelle A, R, $R_1$ et $R_2$ ont la signification déjà indiquée,
que l'on isole et si désiré salifie, soit l'on résuit ledit produit de formule ($I_A$) au niveau de la fonction cétone, pour obtenir un produit de formule ($I_E$) :

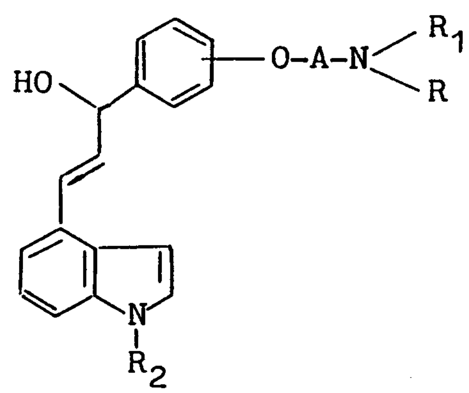

$I_E$

dans laquelle A, R, $R_1$ et $R_2$ ont la signification déjà indiquée,
que ou bien l'on isole, ou bien l'on réduit au niveau de la double liaison de la chaîne aliphatique, pour obtenir un produit de formule ($I_D$) due l'on isole et, si désiré, salifie,
soit l'on réduit ledit produit de formule $I_A$ à la fois au niveau de la fonction cétone et à celui de la double liaison, pour obtenir un produit de formule $I_D$ que l'on isole et, si désiré salifie,
puis soumet si désiré lesdits produits de formule $I_A$, $I_B$, $I_C$, $I_D$
et $I_E$ à l'action d'un agent d'halogénation pour obtenir un produit de formule (VI) :

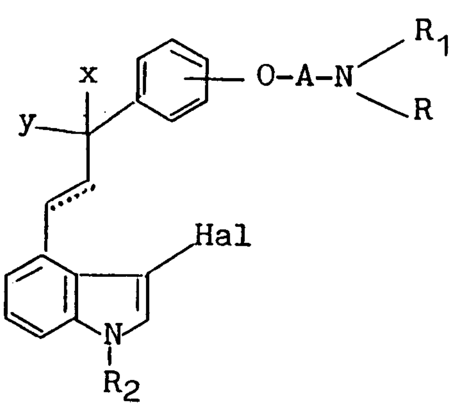

VI

dans laquelle Hal représente un atome de brome ou de chlore, et x, y, A, R, $R_1$, $R_2$ et le pointillé ont la signification déjà indiquée, que l'on soumet à une hydrolyse pour obtenir un produit de formule ($I_F$) :

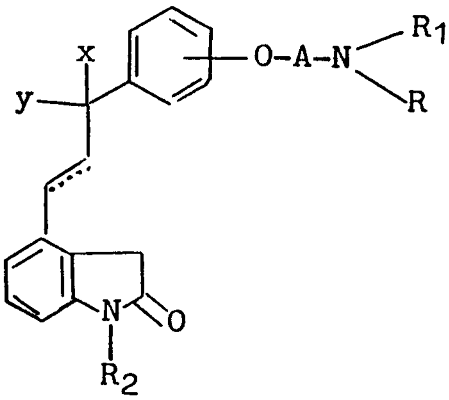

$I_F$

dans laquelle x, y, A, R, $R_1$, $R_2$ et le pointillé ont la signification déjà indiquée, que l'on isole et, si désiré, salifie.

**11.** Médicaments, caractérisés en ce qu'ils sont constitués par les nouveaux dérivés de l'hydroxy alkoxy 4-phénylpropyl indole, tels que définis par la formule (I) de la revendication 1, ainsi que par leurs sels d'addition avec les acides pharmaceutiquement acceptables.

**12.** Médicaments, caractérisés en ce qu'ils sont constitués par les nouveaux dérivés l'hydroxy alkoxy 4-phénylpropyl indole, tels que définis dans l'une des revendications 2 ou 3, ainsi que par leurs sels d'addition avec les acides pharmaceutiquement acceptables.

**13.** Médicaments, caractérisés en ce qu'ils sont constitués par le dérivé de l'hydroxy alkoxy 4-phénylpropyl indole, tel que défini à la revendication 4, ainsi que par leurs sels d'addition avec les acides pharmaceutiquement acceptables.

**14.** Médicaments, caractérisés en ce qu'ils sont constitués par les nouveaux dérivés de l'hydroxy alkoxy 4-phénylpropyl indole, tels que définis dans l'une des revendications 5 ou 6, ainsi que par leurs sels d'addition avec les acides pharmaceutiquement acceptables.

**15.** Médicaments, caractérisés en ce qu'ils sont constitués par le dérivé de l'hydroxy alkoxy 4-phénylpropyl indole, tel que défini à la revendication 7, ainsi que par leurs sels d'addition avec les acides pharmaceutiquement acceptables.

**16.** Compositions pharmaceutiques, caractérisées en ce qu'elles renferment, à titre de principe actif, l'un au moins des médicaments tels que définis à l'une des revendications 11 à 15.

**17.** Les dérivés de formule (IV) :

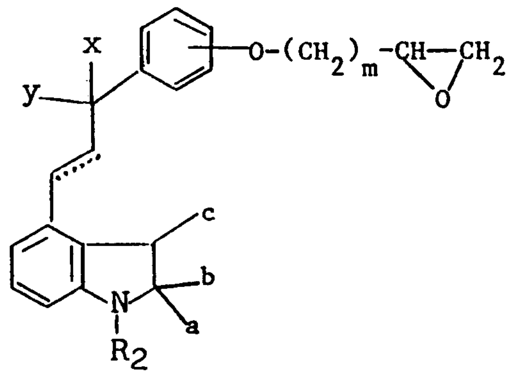

IV

dans laquelle $R_2$, a, b, c, x, y, m et le pointillé ont la signification déjà indiquée, ainsi que les dérivés de formule (VI) :

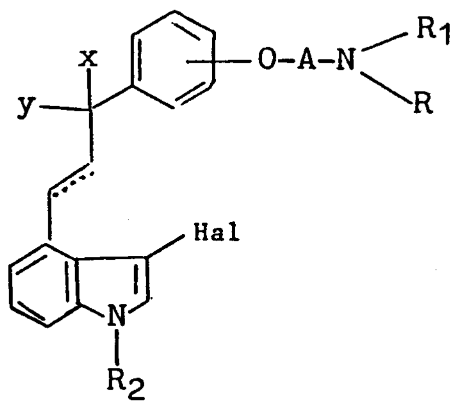

VI

dans laquelle R, $R_1$, $R_2$, x, y, A, Hal et le pointillé ont la signification déjà indiquée.

**Revendications pour l'Etat contractant suivant : AT**

**1.** Procédé de préparation des dérivés de l'hydroxy alkoxy 4-phénylpropyl indole, et de leurs sels d'addition avec les acides minéraux ou organiques, répondant à la formule générale I :

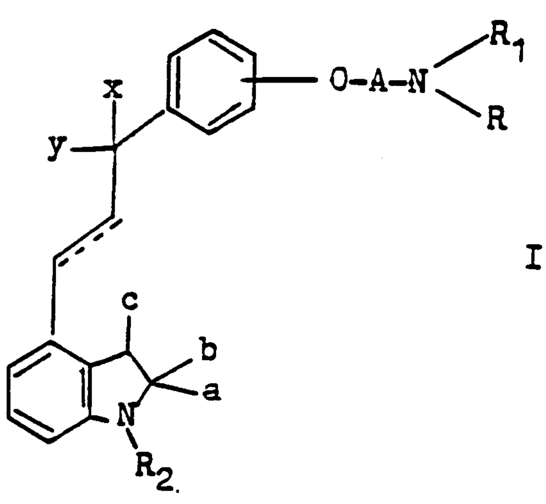

I

dans laquelle R et $R_1$ représentent, indépendamment l'un de l'autre, un atome d'hydrogène, un radical

alkyle linéaire renfermant de 1 à 5 atomes de carbone, un radical alkyle ramifié renfermant de 3 à 5 atomes de carbone, a représente ensemble avec b une fonction oxo, ou représente ensemble avec c une liaison carbone-carbone, b représente un atome d'hydrogène, ou ensemble avec a une fonction oxo, c représente un atome d'hydrogène, ou ensemble avec a représente une liaison carbone-carbone, le pointillé représente la présence éventuelle d'une liaison carbone-carbone, A représente une chaîne

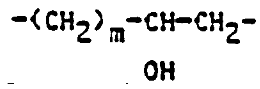

dans laquelle m peut prendre les valeurs 1, 2 ou 3, $R_2$ représente un atome d'hydrogène, un radical alkyle linéaire renfermant de 1 à 5 atomes de carbone ou un radical alkyle ramifié renfermant de 3 à 5 atomes de carbone, x représente un radical hydroxyle, ou un atome d'hydrogène, ou ensemble avec y, une fonction oxo et y représente un atome d'hydrogène ou, ensemble avec x, une fonction oxo, caractérisé en ce que l'on fait réagir un dérivé de formule (II) :

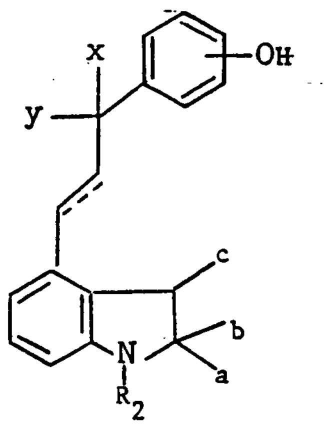

(II)

dans laquelle $R_2$, a, b, c, x, y, et le pointillé ont la signification déjà indiquée, avec un halogénure de formule (III) :

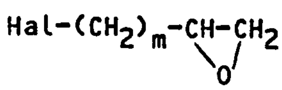

(III)

dans laquelle m a la signification déjà indiquée et Hal représente un atome de chlore, de brome ou d'iode, pour obtenir un produit de formule (IV) :

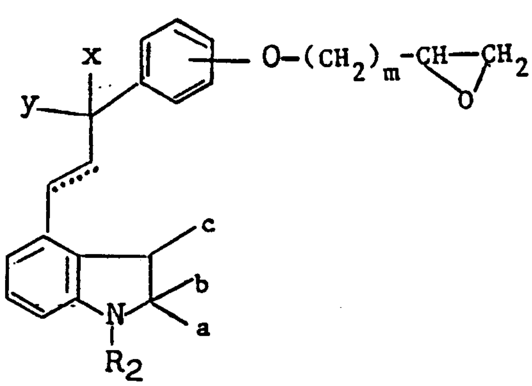

(IV)

dans laquelle $R_2$, a, b, c, x, y, m et le pointillé ont la signification déjà indiquée, que l'on fait réagir avec

une amine de formule (V) :

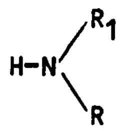

(V)

dans laquelle R et $R_1$ ont la signification déjà indiquée, pour obtenir un produit de formule (I), que l'on isole et, si désiré, salifie.

**2.** Procédé selon la revendication 1, caractérisé en ce que Hal représente un atome de chlore et en ce que la réaction du dérivé de formule (II) avec l'halogénure de formule (III) est utilisée en présence d'une base.

**3.** Procédé selon la revendication 1, caractérisé en ce que l'on fait réagir un produit de formule ($II_A$) :

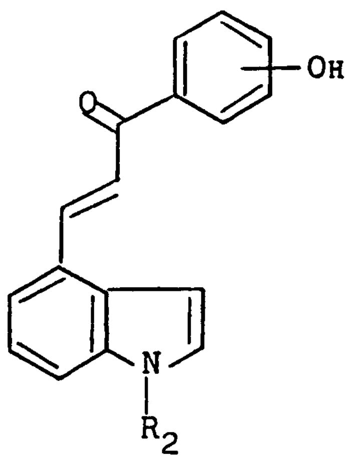

($II_A$)

dans laquelle $R_2$ a la signification déjà indiquée, avec un halogénure de formule (III) :

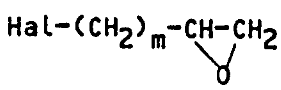

(III)

dans laquelle Hal et m ont la signification déjà indiquée, pour obtenir un produit de formule ($IV_A$) :

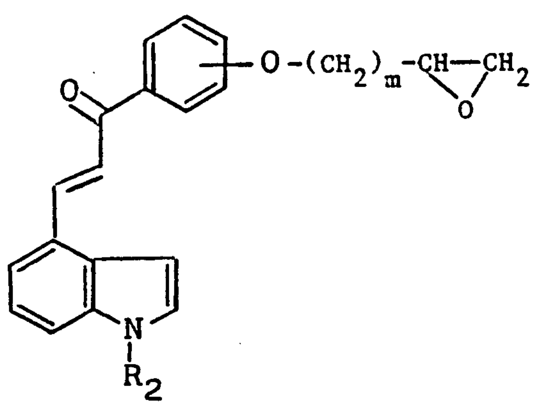

($IV_A$)

dans laquelle $R_2$ et m ont la signification déjà indiquée, que l'on fait réagir avec une amine de formule

(V) :

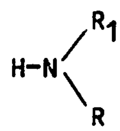

(V)

dans laquelle R et $R_1$ ont la signification déjà indiquée, pour obtenir un produit de formule ($I_A$) :

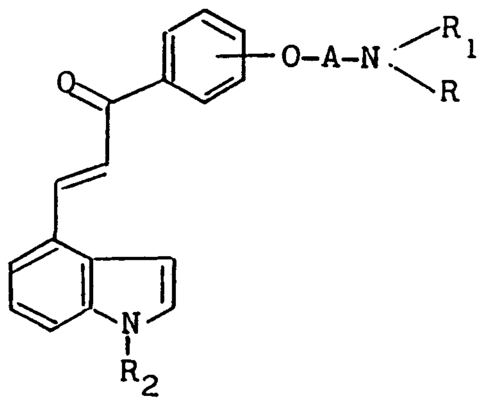

$I_A$

dans laquelle R, $R_1$ et $R_2$ ont la signification déjà indiquée, que
soit l'on réduit au niveau de la double liaison de la chaîne aliphatique, pour obtenir un produit de formule ($I_B$) :

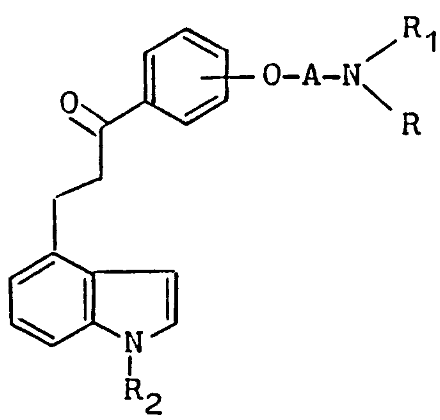

$I_B$

dans laquelle A, R, $R_1$ et $R_2$ ont la signification déjà indiquée, que
ou bien l'on isole et, si désiré salifie, ou bien l'on réduit au niveau de la fonction cétone pour obtenir un produit de formule ($I_C$) :

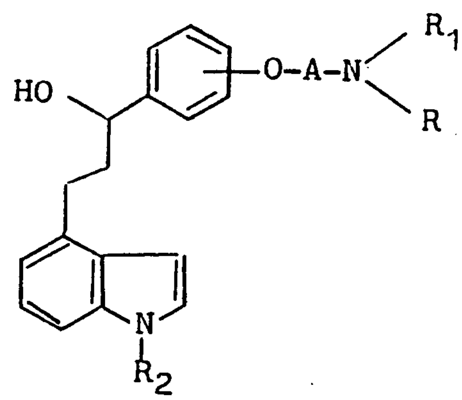

$I_C$

dans laquelle A, R, $R_1$ et $R_2$ ont la signification déjà indiquée, que ou bien l'on isole et, si désiré salifie, ou bien l'on réduit au niveau de la fonction hydroxy, pour obtenir un produit de formule ($I_D$) :

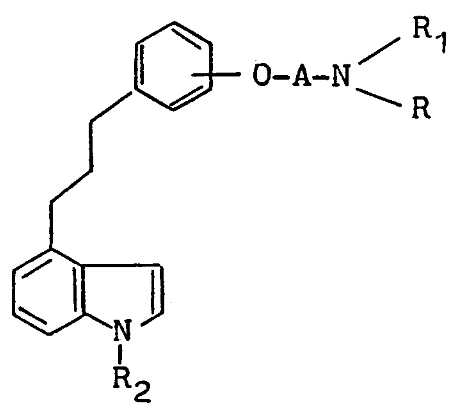

$I_D$

dans laquelle A, R, $R_1$ et $R_2$ ont la signification déjà indiquée, que l'on isole et si désiré salifie, soit l'on réduit ledit produit de formule ($I_A$) au niveau de la fonction cétone, pour obtenir un produit de formule ($I_E$) :

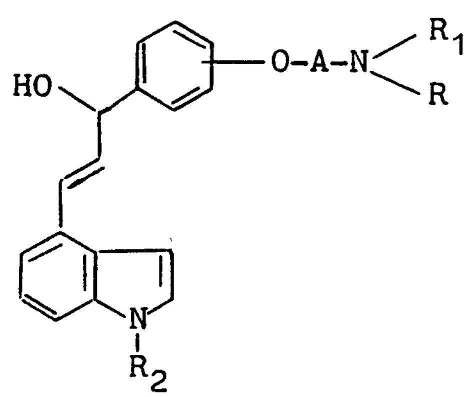

$I_E$

dans laquelle A, R, $R_1$ et $R_2$ ont la signification déjà indiquée, que ou bien l'on isole, ou bien l'on réduit au niveau de la double liaison de la chaîne aliphatique, pour obtenir un produit de formule ($I_D$) que l'on isole et, si désiré, salifie, soit l'on réduit ledit produit de formule $I_A$ à la fois au niveau de la fonction cétone et à celui de la double liaison, pour obtenir un produit de formule $I_D$ que l'on isole et, si désiré salifie, puis soumet si désiré lesdits produits de formule $I_A$, $I_B$, $I_C$, $I_D$ et $I_E$ à l'action d'un agent d'halogénation pour obtenir un produit de formule (VI) :

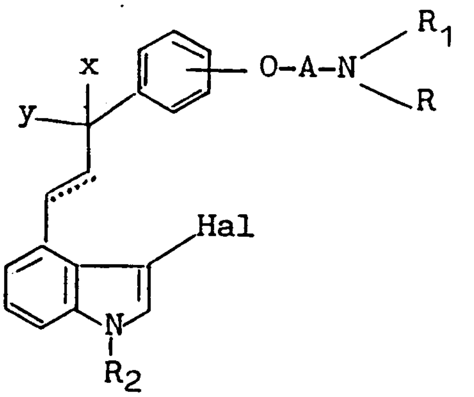

VI

dans laquelle Hal représente un atome de brome ou de chlore, et x, y, A, R, $R_1$, $R_2$ et le pointillé ont la signification déjà indiquée, que l'on soumet à une hydrolyse pour obtenir un produit de formule ($I_F$) :

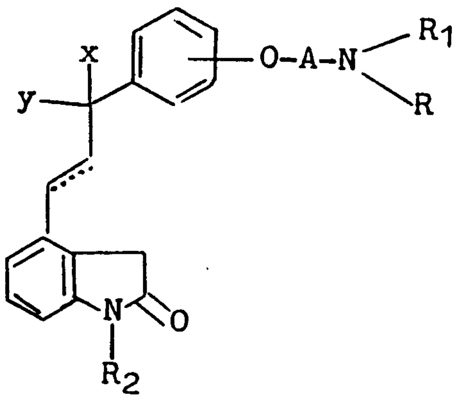

$I_F$

dans laquelle x, y, A, R, $R_1$, $R_2$ et le pointillé ont la signification déjà indiquée, que l'on isole et, si désiré, salifie.

**4.** Procédé selon la revendication 3, caractérisé en ce que :
- la réduction de la double liaison du produit de formule ($I_A$) est effectuée par de l'hydrogène gazeux en présence d'un catalyseur à base de platine ou de palladium, dans un solvant tel qu'un alcanol renfermant de 1 à 5 atomes de carbone, ou par de l'hydrogène gazeux en présence de Nickel de Raney dans un solvant tel que l'acétate d'éthyle, ou encore par action du sodium dans l'ammoniac dans un solvant tel que le tétrahydrofuranne, le temps du contact étant de moins de 3 heures et, de préférence, d'environ 1 heure ;
- la réduction de la fonction cétone du produit de formule ($I_B$) est effectuée par un borohydrure ou un cyanoborohydrure alcalin ;
- la réduction de la fonction hydroxy du produit de formule ($I_C$) est effectuée de préférence par action du sodium dans l'ammoniac ;
- la réduction de la fonction cétone du produit de formule ($I_A$) est effectuée par le complexe formé entre un borohydrure alcalin, de préférence le borohydrure de sodium, et la pyridine ;
- la réduction de la double liaison du produit de formule ($I_E$) est effectuée de préférence par le sodium dans l'ammoniac ;
- la réduction simultanée de la fonction cétone et de la double liaison du produit de formule ($I_A$) est effectuée de préférence par le sodium dans l'ammoniac ;
- Hal représente un atome de chlore.

**5.** Procédé selon l'une quelconque des revendications 1 à 4, caractérisé en ce que dans le produit de formule (II) ou ($II_A$), $R_2$ représente un atome d'hydrogène et en ce que dans le produit de formule (V), $R_1$ représente un atome d'hydrogène.

**6.** Procédé selon l'une quelconque des revendications 1 à 4, caractérisé en ce que dans le produit de formule (II) ou ($II_A$), $R_2$ représente un atome d'hydrogène et en ce que dans le produit de formule (V), $R_1$ représente un radical propyle.

**7.** Procédé selon la revendication 5, caractérisé en ce que l'on prépare la 1-/2-/3-/(1,1-diméthyléthyl) amino/ 2-hydroxypropoxy/ phényl/ 3-(1H-indol-4-yl) 1-propanone ainsi que ses sels d'addition avec les acides minéraux ou organiques.

**8.** Procédé selon la revendication 6, caractérisé en ce que l'on prépare la 1,3-dihydro 4-/3-/2-/3-propylamino/ 2-hydroxypropoxy/ phényl/ propyl/ 2H-indol-2-one, ainsi que ses sels d'addition avec les acides minéraux ou organiques.

**Claims**

**Claims for the following Contracting States : BE, CH, DE, FR, GB, IT, LI, LU, NL, SE**

**1.** The derivatives of hydroxy alkoxy 4-phenylpropyl indole as well as their addition salts with mineral or organic acids, characterized in that they correspond to general formula (I):

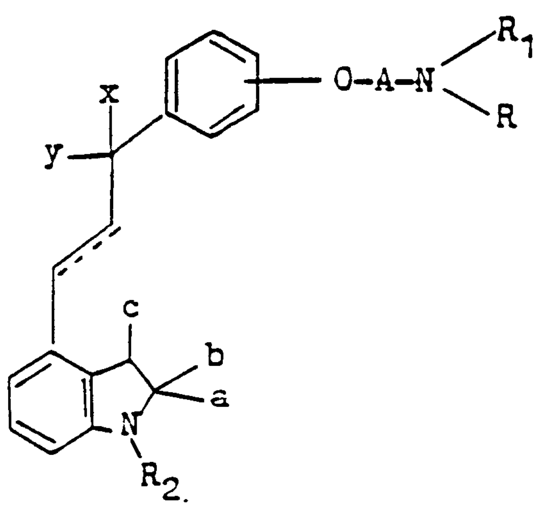

(I)

in which R and $R_1$ represent, independently of each other, a hydrogen atom, a linear alkyl radical containing 1 to 5 carbon atoms, a branched alkyl radical containing 3 to 5 carbon atoms, a represents together with b an oxo function, or represent together with c a carbon-carbon bond, b represents a hydrogen atom, or together with a an oxo function, c represents a hydrogen atom, or together with a represents a carbon-carbon bond, the dotted line represents the optional presence of a carbon-carbon bond, A represents a

$$-(CH_2)_m-\underset{OH}{\underset{|}{C}H}-CH_2-$$

chain in which m has the value 1, $R_2$ represents a hydrogen atom, a linear alkyl radical containing 1 to 5 carbon atoms or a branched alkyl radical containing 3 to 5 carbon atoms, x represents a hydroxyl radical, or a hydrogen atom, or together with y, an oxo function and y represents a hydrogen atom or, together with x, an oxo function.

**2.** The derivatives as defined by formula (I) of claim 1, as well as their addition salts with mineral or organic acids, characterized in that a and c represent together a carbon-carbon bond.

**3.** The derivatives according to claim 2, as well as their addition salts with mineral or organic acids, characterized in that $R_1$ and $R_2$ represent a hydrogen atom.

4. The derivative of formula (I) according to claim 1 the name of which follows:
   - 1-/2-/3-/(1,7-dimethylethyl) amino/ 2-hydroxypropoxy/ phenyl/ 3-(1H-indol-4-yl) 1-propanone, as well as its addition salts with mineral or organic acids.

5. The derivatives as defined by formula (I) of claim 1, as well as their addition salts with mineral or organic acids, characterized in that a and b represent together a ketone function.

6. The derivatives according to claim 5, as well as their addition salts with mineral or organic acids, characterized in that $R_1$ represents a propyl radical and $R_2$ represents a hydrogen atom.

7. The derivative of formula (I) according to claim 1 the name of which follows:
   - 1,3-dihydro 4-/3-/2-/3-propylamino/ 2-hydroxypropoxy/ phenyl/ propyl/ 2H-indol-2-one, as well as its addition salts with mineral or organic acids.

8. Preparation process for the derivatives as defined by formula (I) of claim 1, as well as their salts, characterized in that a derivative of formula (II):

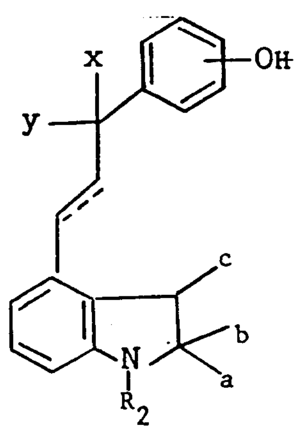

(II)

in which $R_2$, a, b, c, x, y and the dotted line have the meaning already indicated, is reacted with a halide of formula (III):

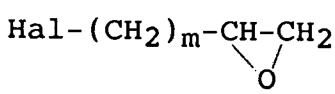

(III)

in which m has the meaning already indicated and Hal represents a chlorine, bromine or iodine atom, in order to obtain a product of formula (IV):

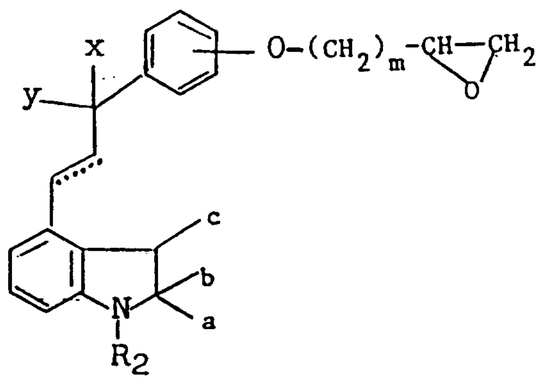

(IV)

in which $R_2$, a, b, c, x, y, m and the dotted line have the meaning already indicated, which is reacted with an amine of formula (V):

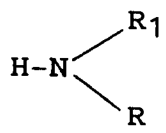

(V)

in which R and $R_1$ have the meaning already indicated, in order to obtain a product of formula (I), which is isolated and, if desired, salified.

**9.** Process according to claim 8, characterized in that Hal represents a chlorine atom and in that the reaction of the derivative of formula (II) with the halide of formula (III) takes place in the presence of a base.

**10.** Process according to claim 8, characterized in that a product of formula ($II_A$):

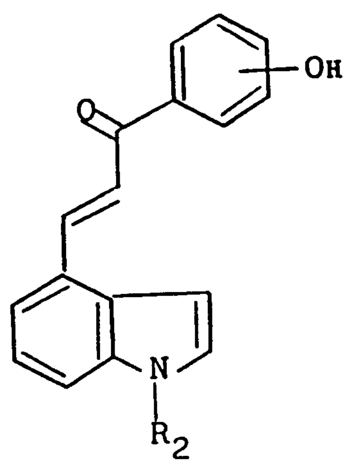

($II_A$)

in which $R_2$ has the meaning already indicated, is reacted with a halide of formula (III):

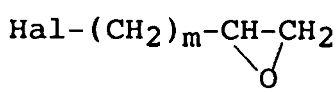

(III)

in which Hal and m have the meaning already indicated, in order to obtain a product of formula ($IV_A$):

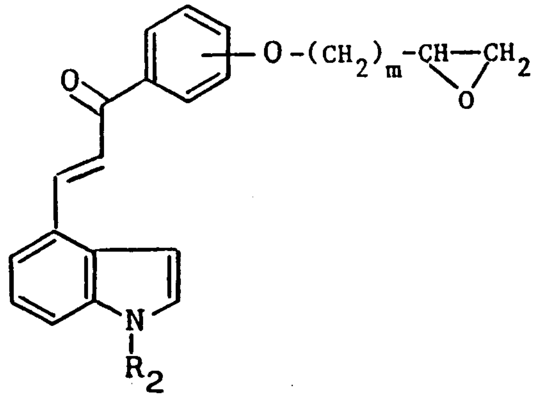

($IV_A$)

in which $R_2$ and m have the meaning already indicated, which is reacted with an amine of formula (V):

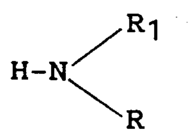

(V)

in which R and $R_1$ have the meaning already indicated, in order to obtain a product of formula ($I_A$):

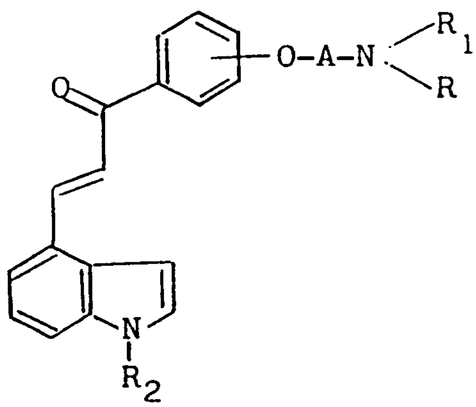

($I_A$)

in which R, $R_1$ and $R_2$ have the meaning already indicated, which either is reduced at the level of the double bond of the aliphatic chain, in order to obtain a product of formula ($I_B$):

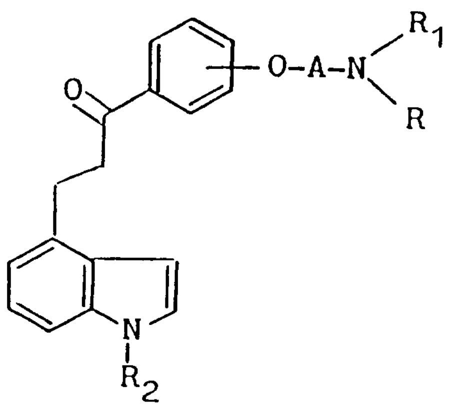

($I_B$))

in which A, R, $R_1$ and $R_2$ have the meaning already indicated, which either is isolated and, if desired, salified, or is reduced at the level of the ketone function in order to obtain a product of formula ($I_C$):

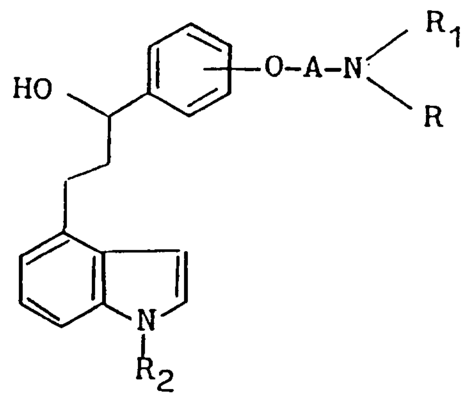

($I_C$)

in which A, R, $R_1$ and $R_2$ have the meaning already indicated, which either is isolated and, if desired, salified, or reduced at the level of the hydroxy function, in order to obtain a product of formula ($I_D$):

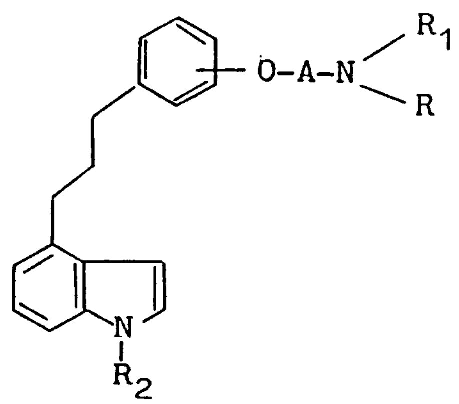

($I_D$)

in which A, R, $R_1$ and $R_2$ have the meaning already indicated, which is isolated and, if desired, salified, or the said product of formula ($I_A$) is reduced at the level of the ketone function, in order to obtain a product of formula ($I_E$):

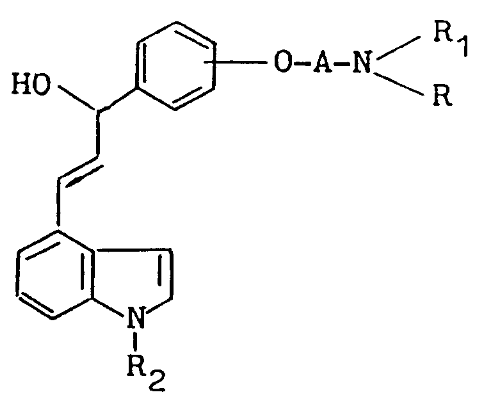

($I_E$)

in which A, R, $R_1$ and $R_2$ have the meaning already indicated, which either is isolated, or reduced at the level of the double bond of the aliphatic chain, in order to obtain a product of formula ($I_D$) which is isolated and, if desired, salified,

or the said product of formula $I_A$ is reduced at the level of the ketone function and at the level of the double bond, in order to obtain a product of formula $I_D$ which is isolated and, if desired, salified,

then, if desired, the said products of formulae $I_A$, $I_B$, $I_C$, $I_D$ and $I_E$ are subjected to the action of a halogenation agent in order to obtain a product of formula (VI):

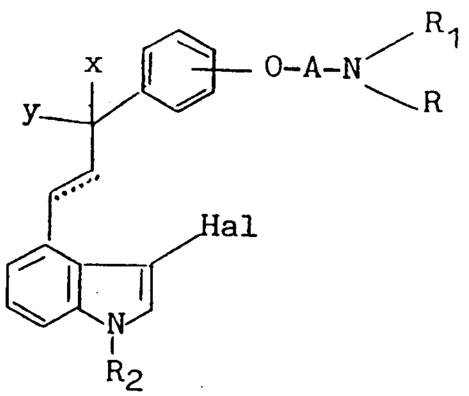

(VI)

in which Hal represents a bromine or chlorine atom, and x, y, A, R, $R_1$, $R_2$ and the dotted line have the meaning already indicated, which is subjected to a hydrolysis in order to obtain a product of formula ($I_F$):

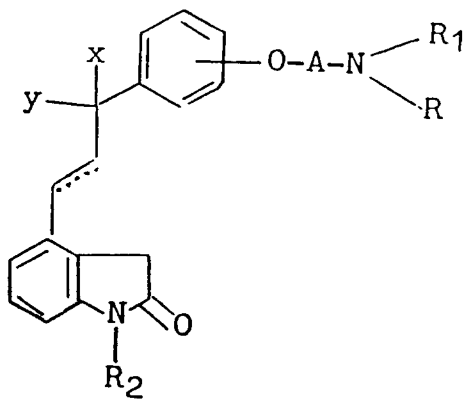

($I_F$)

in which x, y, A, R, $R_1$, $R_2$ and the dotted line have the meaning already indicated, which is isolated and, if desired, salified.

**11.** Medicaments, characterized in that they are constituted by the new derivatives of hydroxy alkoxy 4-phenylpropyl indole, as defined by formula (I) of claim 1, as well as by their addition salts with pharmaceutically acceptable acids.

**12.** Medicaments, characterized in that they are constituted by the new derivatives of hydroxy alkoxy 4-phenylpropyl indole, as defined in one of claims 2 or 3, as well as by their addition salts with pharmaceutically acceptable acids.

**13.** Medicaments, characterized in that they are constituted by the derivative of hydroxy alkoxy 4-phenylpropyl indole, as defined in claim 4, as well as their addition salts with pharmaceutically acceptable acids.

**14.** Medicaments, characterized in that they are constituted by the new derivatives of hydroxy alkoxy 4-phenylpropyl indole, as defined in one of claims 5 or 6, as well as by their addition salts with pharmaceutically acceptable acids.

**15.** Medicaments, characterized in that they are constituted by the derivative of hydroxy alkoxy 4-phenylpropyl indole, as defined in claim 7, as well as by their addition salts with pharmaceutically acceptable acids.

**16.** Pharmaceutical compositions, characterized in that they contain, as active ingredient, at least one of the medicaments as defined in one of claims 11 to 15.

**17.** The derivatives of formula (IV):

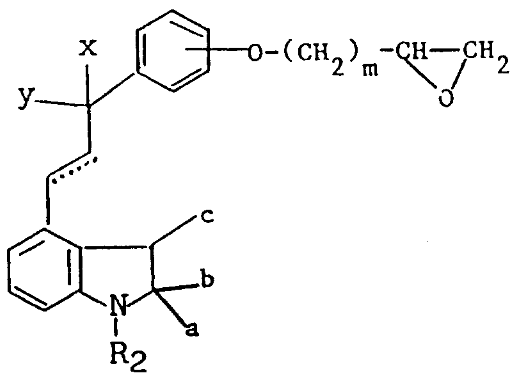

(IV)

in which $R_2$, a, b, c, x, y, m, and the dotted line have the meaning already indicated, as well as the derivatives of formula (VI):

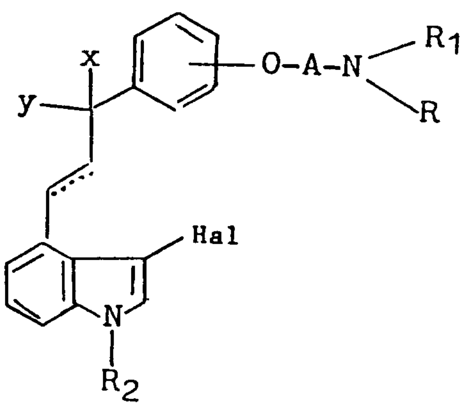

(VI)

in which R, $R_1$, $R_2$, x, y, A, Hal and the dotted line have the meaning already indicated.

**Claims for the following Contracting State : AT**

**1.** Preparation process for the derivatives of hydroxy alkoxy 4-phenylpropyl indole as well as their addition salts with mineral or organic acids, corresponding to general formula (I):

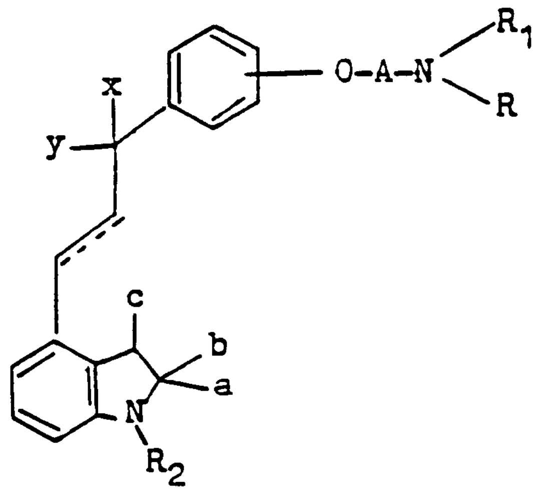

(I)

in which R and $R_1$ represent, independently of each other, a hydrogen atom, a linear alkyl radical containing 1 to 5 carbon atoms, a branched alkyl radical containing 3 to 5 carbon atoms, a represents together with b an oxo function, or represent together with c a carbon-carbon bond, b represents a hydrogen atom, or together with a an oxo function, c represents a hydrogen atom, or together with a represents a carbon-carbon bond, the dotted line represents the optional presence of a carbon-carbon bond, A represents a

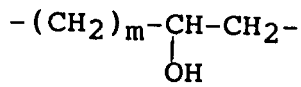

chain in which m can have the values 1, 2 or 3, $R_2$ represents a hydrogen atom, a linear alkyl radical containing 1 to 5 carbon atoms or a branched alkyl radical containing 3 to 5 carbon atoms, x represents a hydroxyl radical, or a hydrogen atom, or together with y, an oxo function and y represents a hydrogen atom or, together with x, an oxo function, characterized in that a derivative of formula (II):

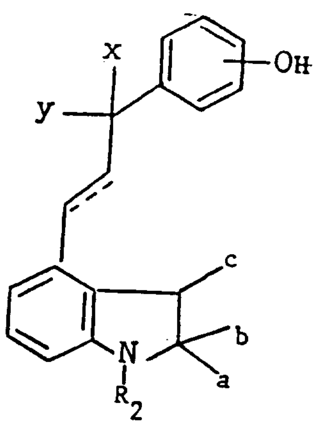

(II)

in which $R_2$, a, b, c, x, y and the dotted line have the meaning already indicated, is reacted with a halide of formula (III):

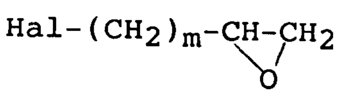

(III)

in which m has the meaning already indicated and Hal represents a chlorine, bromine or iodine atom, in order to obtain a product of formula (IV):

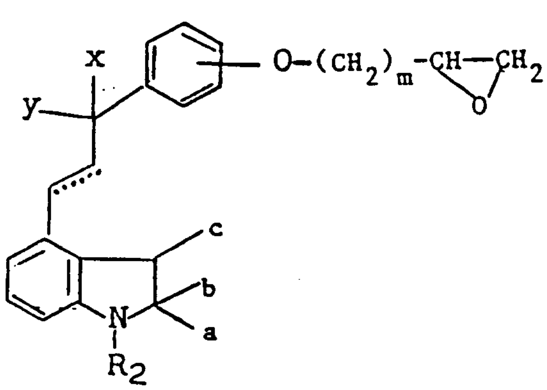

(IV)

in which $R_2$, a, b, c, x, y, m and the dotted line have the meaning already indicated, which is reacted

with an amine of formula (V):

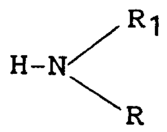

(V)

in which R and $R_1$ have the meaning already indicated, in order to obtain a product of formula (I), which is isolated and, if desired, salified.

**2.** Process according to claim 1, characterized in that Hal represents a chlorine atom and in that the reaction of the derivative of formula (II) with the halide of formula (III) takes place in the presence of a base.

**3.** Process according to claim 1, characterized in that a product of formula ($II_A$):

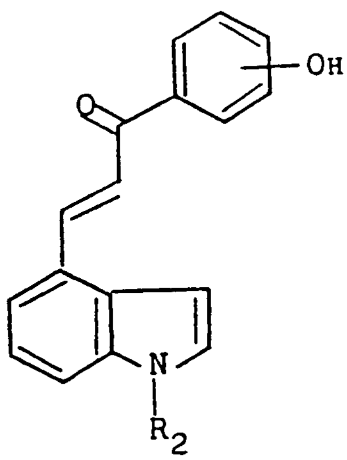

($II_A$)

in which $R_2$ has the meaning already indicated, is reacted with a halide of formula (III):

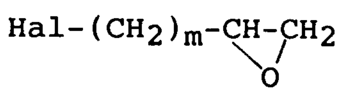

(III)

in which Hal and m have the meaning already indicated, in order to obtain a product of formula ($IV_A$):

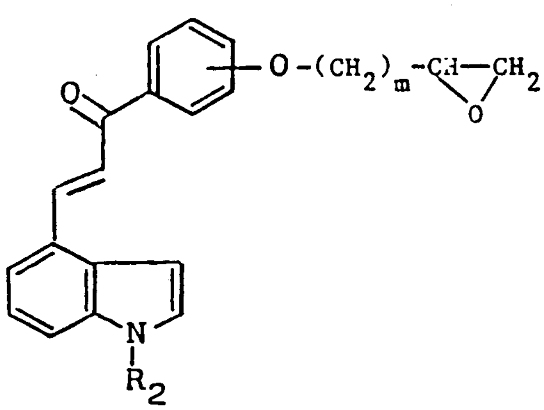

($IV_A$)

in which $R_2$ and m have the meaning already indicated, which is reacted with an amine of formula (V):

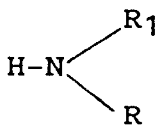

(V)

in which R and $R_1$ have the meaning already indicated, in order to obtain a product of formula ($I_A$):

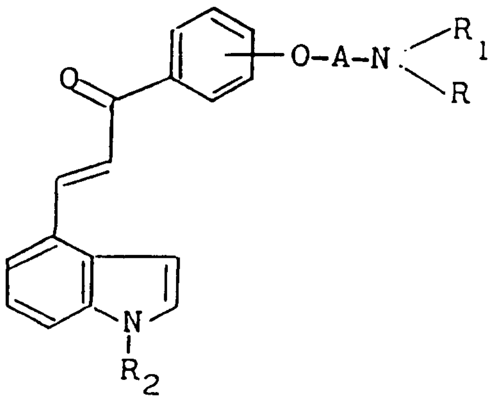

($I_A$)

in which R, $R_1$ and $R_2$ have the meaning already indicated, which either is reduced at the level of the double bond of the aliphatic chain, in order to obtain a product of formula ($I_B$):

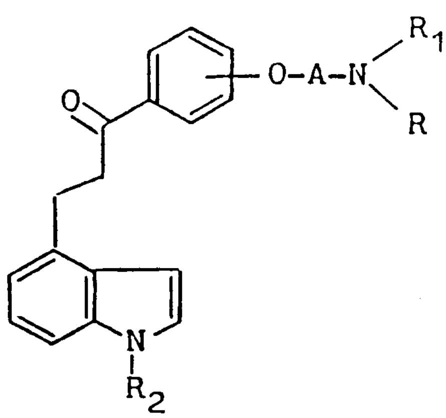

($I_B$))

in which A, R, $R_1$ and $R_2$ have the meaning already indicated, which either is isolated and, if desired, salified, or is reduced at the level of the ketone function in order to obtain a product of formula ($I_C$):

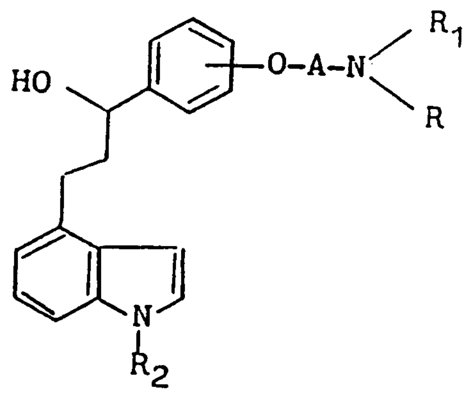

(I$_C$)

in which A, R, $R_1$ and $R_2$ have the meaning already indicated, which either is isolated and, if desired, salified, or reduced at the level of the hydroxy function, in order to obtain a product of formula ($I_D$):

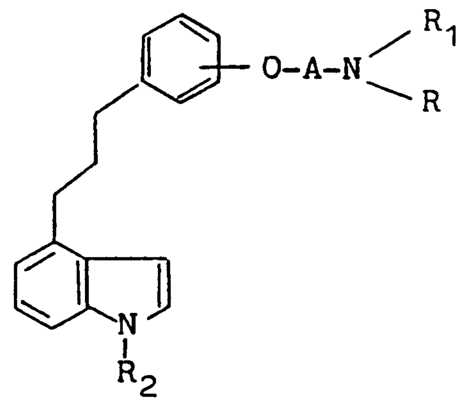

(I$_D$)

in which A, R, $R_1$ and $R_2$ have the meaning already indicated, which is isolated and, if desired, salified, or the said product of formula ($I_A$) is reduced at the level of the ketone function, in order to obtain a product of formula ($I_E$):

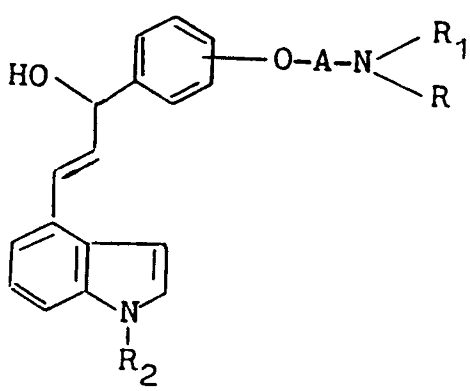

(I$_E$)

in which A, R, $R_1$ and $R_2$ have the meaning already indicated, which either is isolated, or reduced at the level of the double bond of the aliphatic chain, in order to obtain a product of formula ($I_D$) which is isolated and, if desired, salified,

or the said product of formula $I_A$ is reduced at the level of the ketone function and at the level of the double bond, in order to obtain a product of formula $I_D$ which is isolated and, if desired, salified,

then, if desired, the said products of formulae $I_A$, $I_B$, $I_C$, $I_D$ and $I_E$ are subjected to the action of a halogenation agent in order to obtain a product of formula (VI):

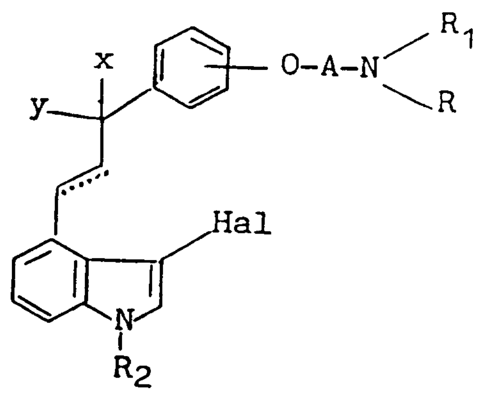

(VI)

in which Hal represents a bromine or chlorine atom, and x, y, A, R, $R_1$, $R_2$ and the dotted line have the meaning already indicated, which is subjected to a hydrolysis in order to obtain a product of formula ($I_F$):

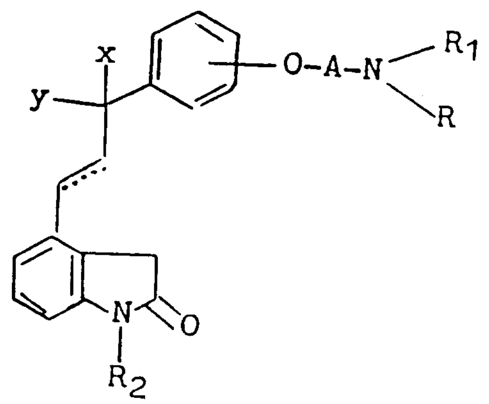

($I_F$)

in which x, y, A, R, $R_1$, $R_2$ and the dotted line have the meaning already indicated, which is isolated and, if desired, salified.

**4.** Process according to claim 3, characterized in that:
- the reduction of the double bond of the product of formula ($I_A$) is carried out by gaseous hydrogen in the presence of a catalyst based on platinum or palladium, in a solvent such as an alkanol containing 1 to 5 carbon atoms, or by gaseous hydrogen in the presence of Raney Nickel in a solvent such as ethyl acetate, or also by the action of sodium in ammonia in a solvent such as tetrahydrofuran, the contact time being less than 3 hours and, preferably, about one hour;
- the reduction of the ketone function of the product of formula ($I_B$) is carried out by an alkaline borohydride or cyanoborohydride;
- the reduction of the hydroxy function of the product of formula ($I_C$) is preferably carried out by the action of sodium in ammonia;
- the reduction of the ketone function of the product of formula ($I_A$) is carried out by the complex formed between an alkaline borohydride, preferably sodium borohydride, and pyridine;
- the reduction of the double bond of the product of formula ($I_E$) is preferably carried out by sodium in ammonia;
- the simultaneous reduction of the ketone function and the double bond of the product of formula ($I_A$) is preferably carried out by sodium in ammonia;
- Hal represents a chlorine atom.

**5.** Process according to one of claims 1 to 4, characterized in that in the product of formula (II) or ($II_A$), $R_2$ represents a hydrogen atom and in that in the product of formula (V), $R_1$ represents a hydrogen atom.

6. Process according to one of claims 1 to 4, characterized in that in the product of formula (II) or ($II_A$), $R_2$ represents a hydrogen atom and in that in the product of formula (V), $R_1$ represents a propyl radical.

7. Process according to claim 5, characterized in that 1-/2-/3-/(1,1-dimethylethyl) amino/ 2-hydroxypropoxy/ phenyl/ 3-(1H-indol-4-yl) 1-propanone is prepared, as well as its addition salts with mineral or organic acids.

8. Process according to claim 6, characterized in that 1,3-dihydro 4-/3-/2-/3-propylamino/ 2-hydroxypropoxy/ phenyl/ propyl/ 2H-indol-2-one is prepared, as well as its addition salts with mineral or organic acids.

**Patentansprüche**
**Patentansprüche für folgende Vertragsstaaten : BE, CH, DE, FR, GB, IT, LI, LU, NL, SE**

1. Hydroxyalkoxy-4-phenylpropylindol-Derivate sowie deren Additionssalze mit Mineral- oder organischen Säuren, dadurch gekennzeichnet, daß sie der allgemeinen Formel (I)

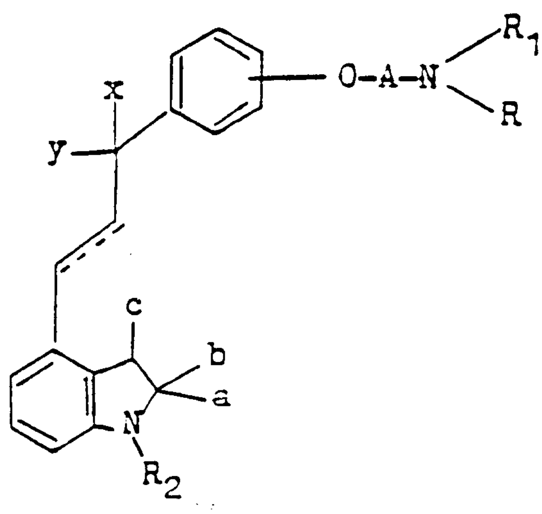

I

entsprechen, worin R und $R_1$, unabhängig voneinander, ein Wasserstoffatom, einen linearen Alkylrest mit 1 bis 5 Kohlenstoffatomen, einen verzweigten Alkylrest mit 3 bis 5 Kohlenstoffatomen bedeuten, a zusammen mit b eine Oxofunktion wiedergibt oder gemeinsam mit c eine Kohlenstoff-Kohlenstoff-Bindung darstellt, b ein Wasserstoffatom bedeutet oder gemeinsam mit a eine Oxofunktion wiedergibt, c ein Wasserstoffatom bedeutet oder gemeinsam mit a eine Kohlenstoff-Kohlenstoff-Bindung wiedergibt, wobei die gestrichelte Linie die etwaige Anwesenheit einer Kohlenstoff-Kohlenstoff-Bindung anzeigt, A für eine Kette

$$-(CH_2)_m-\underset{OH}{CH}-CH_2-$$

steht, worin m den Wert 1 besitzt, $R_2$ ein Wasserstoffatom, einen linearen Alkylrest mit 1 bis 5 Kohlenstoffatomen oder einen verzweigten Alkylrest mit 3 bis 5 Kohlenstoffatomen wiedergibt, x eine Hydroxylgruppe oder ein Wasserstoffatom bedeutet oder gemeinsam mit y eine Oxofunktion wiedergibt, und y ein Wasserstoffatom oder gemeinsam mit x eine Oxofunktion bedeutet.

2. Derivate der Formel (I) gemäß Anspruch 1 sowie deren Additionssalze mit Mineral- oder organischen Säuren, dadurch gekennzeichnet, daß a und c gemeinsam eine Kohlenstoff-Kohlenstoff-Bindung wiedergeben.

**3.** Derivate gemäß Anspruch 2 sowie deren Additionssalze mit Mineral- oder organischen Säuren, dadurch gekennzeichnet, daß $R_1$ und $R_2$ ein Wasserstoffatom bedeuten.

**4.** Das Derivat der Formel (I) gemäß Anspruch 1 mit der folgenden Bezeichnung: 1-[2-[3-[(1,1-Dimethylethyl)-amino]-2-hydroxypropoxy]-phenyl]-3-(1H-indol-4-yl)-1-propanon sowie dessen Additionssalze mit Mineral- oder organischen Säuren.

**5.** Derivate der Formel (I) gemäß Anspruch 1 sowie deren Additionssalze mit Mineral- oder organischen Säuren, dadurch gekennzeichnet, daß a und b gemeinsam eine Ketonfunktion wiedergeben.

**6.** Derivate gemäß Anspruch 5 sowie deren Additionssalze mit Mineral- oder organischen Säuren, dadurch gekennzeichnet, daß $R_1$ einen Propylrest darstellt, und $R_2$ für ein Wasserstoffatom steht.

**7.** Derivat der Formel (I) gemäß Anspruch 1 mit der folgenden Bezeichnung: 1,3-Dihydro-4-[3-[2-[3-propylamino]-2-hydroxypropoxy]-phenyl]-propyl]-2H-indol-2-on sowie dessen Additionssalze mit Mineral- oder organischen Säuren.

**8.** Verfahren zur Herstellung der Derivate der Formel (I) gemäß Anspruch 1 sowie von deren Salzen, dadurch gekennzeichnet, daß man ein Derivat der Formel (II)

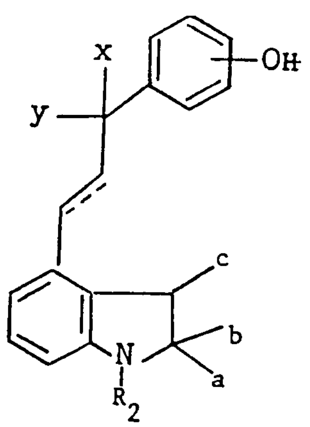

(II)

worin $R_2$, a, b, c, x, y und die gestrichelte Linie die angegebene Bedeutung besitzen, mit einem Halogenid der Formel (III)

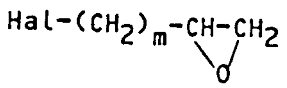

(III)

worin m die angegebene Bedeutung besitzt, und Hal für ein Chlor-, Brom- oder Jodatom steht, umsetzt, um zu einem Produkt der Formel (IV)

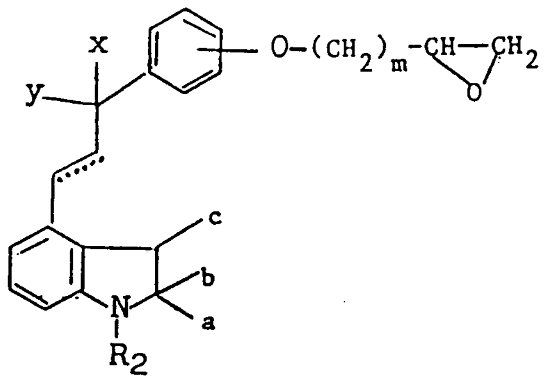

(IV)

zu gelangen, worin $R_2$, a, b, c, x, y, m und die gestrichelte Linie die angegebene Bedeutung besitzen, welches man mit einem Amin der Formel (V)

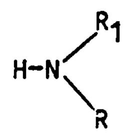

(V)

worin R und $R_1$ die angegebene Bedeutung besitzen, umsetzt, um zu einem Produkt der Formel (I) zu gelangen, welches man isoliert und gewünschtenfalls in ein Salz überführt.

**9.** Verfahren gemäß Anspruch 8, dadurch gekennzeichnet, daß Hal für ein Chloratom steht, und daß die Umsetzung des Derivats der Formel (II) mit dem Halogenid der Formel (III) in Gegenwart einer Base durchgeführt wird.

**10.** Verfahren gemäß Anspruch 8, dadurch gekennzeichnet, daß man ein Produkt der Formel ($II_A$)

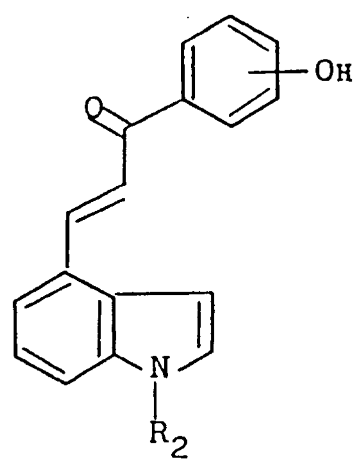

($II_A$)

worin $R_2$ die angegebene Bedeutung besitzt, mit einem Halogenid der Formel (III)

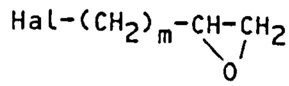

(III)

worin Hal und m die angegebene Bedeutung besitzen, umsetzt, um zu einem Produkt der Formel ($IV_A$)

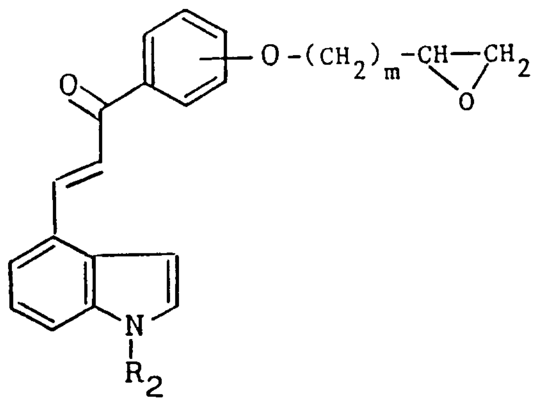

$(IV_A)$

zu gelangen, worin $R_2$ und m die angegebene Bedeutung besitzen,welches man mit einem Amin der Formel (V)

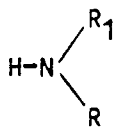

(V)

worin R und $R_1$ die angegebene Bedeutung besitzen, umsetzt, um zu einem Produkt der Formel ($I_A$)

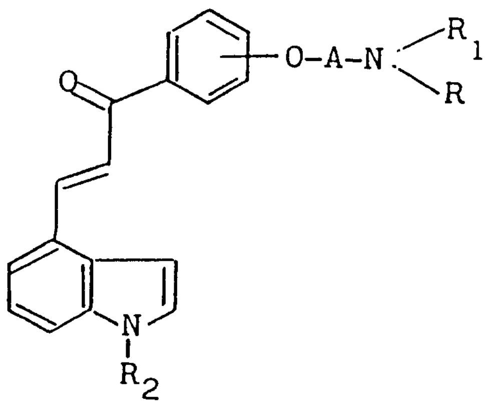

$I_A$

zu gelangen, worin R, $R_1$ und $R_2$ die angegebene Bedeutung besitzen, welches man
entweder im Hinblick auf die Doppelbindung der aliphatischen Kette reduziert, um zu einem Produkt der Formel ($I_B$)

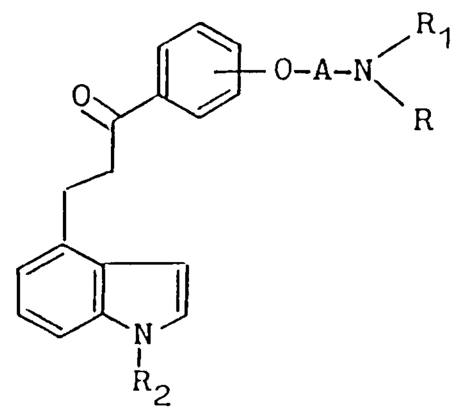

$I_B$

worin A, R, $R_1$ und $R_2$ die angegebene Bedeutung besitzen, zu gelangen, welches man entweder isoliert und gewünschtenfalls in ein Salz überführt oder im Hinblick auf die Ketonfunktion reduziert, um zu einem Produkt der Formel ($I_C$)

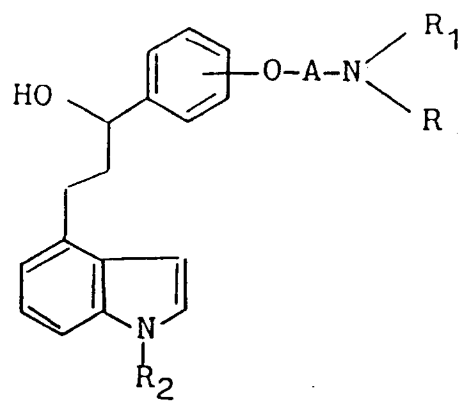

$I_C$

worin A, R, $R_1$ und $R_2$ die angegebene Bedeutung besitzen, zu gelangen, welches man entweder isoliert und gewünschtenfalls in ein Salz überführt oder das man im Hinblick auf die Hydroxyfunktion reduziert, um zu einem Produkt der Formel ($I_D$)

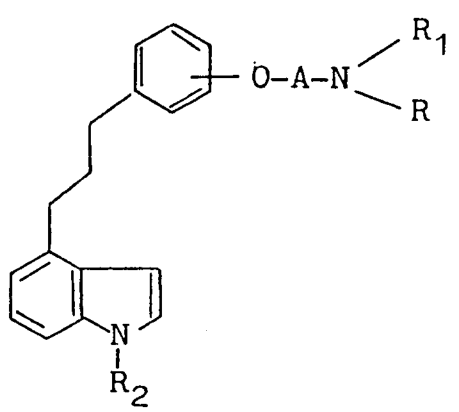

$I_D$

worin A, R, $R_1$ und $R_2$ die angegebene Bedeutung besitzen, zu gelangen, welches man isoliert und gewünschtenfalls in ein Salz überführt, oder das Produkt der Formel ($I_A$) im Hinblick auf die Ketonfunktion reduziert, um zu einem Produkt der Formel ($I_E$)

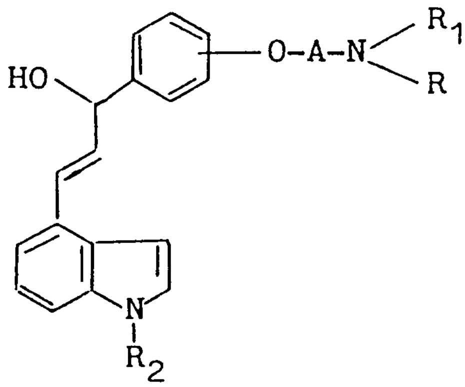

$I_E$

worin A, R, $R_1$ und $R_2$ die angegebene Bedeutung besitzen, zu gelangen, welches man isoliert oder im Hinblick

auf die Doppelbindung der aliphatischen Kette reduziert, um zu einem Produkt der Formel ($I_D$) zu gelangen, welches man isoliert und gewünschtenfalls in ein Salz überführt,

oder das Produkt der Formel ($I_A$) gleichzeitig im Hinblick auf die Ketonfunktion und auf die Doppelbindung reduziert, um zu einem Produkt der Formel ($I_D$) zu gelangen, welches man isoliert und gewünschtenfalls in ein Salz überführt, wonach man gewünschtenfalls diese Produkte der Formel ($I_A$), ($I_B$), ($I_C$), ($I_D$) und ($I_E$) der Einwirkung eines Halogenierungsmittels unterzieht, um zu einem Produkt der Formel (VI)

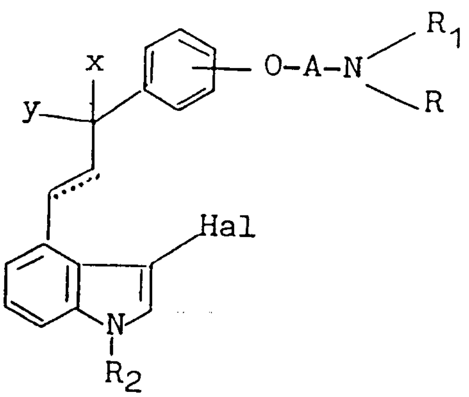

VI

worin Hal für ein Brom- oder Chloratom steht, und x, y und A, R, $R_1$, $R_2$ und die gestrichelte Linie die angegebene Bedeutung besitzen, zu gelangen, welches man einer Hydrolyse unterzieht,um zu einem Produkt der Formel ($I_F$)

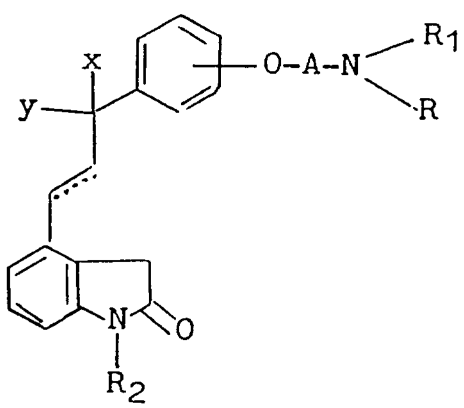

$I_F$

worin x, y, A, $R_1$, $R_2$ und die gestrichelte Linie die angegebene Bedeutung besitzen, zu gelangen, welches man isoliert und gewünschtenfalls in ein Salz überführt.

**11.** Arzneimittel, dadurch gekennzeichnet, daß sie aus den neuen Hydroxyalkoxy-4-phenylpropylindol-Derivaten der Formel (I) gemäß Anspruch 1 sowie aus deren Additionssalzen mit pharmazeutisch verträglichen Säuren bestehen.

**12.** Arzneimittel, dadurch gekennzeichnet, daß sie aus den neuen Hydroxyalkoxy-4-phenylpropylindol-Derivaten, wie in einem der Ansprüche 2 oder 3 definiert, sowie aus deren Additionssalzen mit pharmazeutisch verträglichen Säuren bestehen.

**13.** Arzneimittel, dadurch gekennzeichnet, daß sie aus dem Hydroxyalkoxy-4-phenylpropylindol-Derivat gemäß Anspruch 4 sowie aus deren Additionssalzen mit pharmazeutisch verträglichen Säuren bestehen.

**14.** Arzneimittel, dadurch gekennzeichnet, daß sie aus den neuen Hydroxyalkoxy-4-phenylpropylindol-Derivaten gemäß einem der Ansprüche 5 oder 6 sowie aus deren Additionssalzen mit pharmazeutisch verträglichen Säuren bestehen.

**15.** Arzneimittel, dadurch gekennzeichnet, daß sie aus dem Hydroxyalkoxy-4-phenylpropylindol-Derivat gemäß Anspruch 7 sowie aus deren Additionssalzen mit pharmazeutisch verträglichen Säuren bestehen.

**16.** Pharmazeutische Zusammensetzungen, dadurch gekennzeichnet, daß sie als Wirkstoff zumindest eines der Arzneimittel gemäß einem der Ansprüche 11 bis 15 enthalten.

**17.** Derivate der Formel (IV)

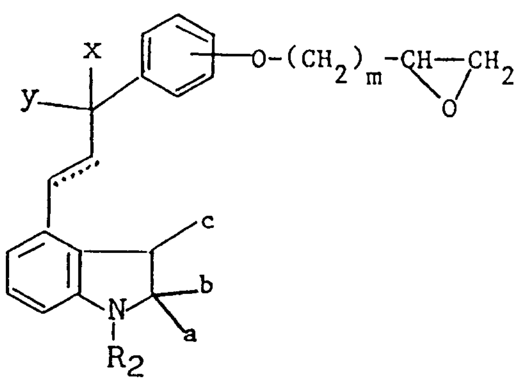

IV

worin $R_2$, a, b, c, x, y, m und die gestrichelte Linie die angegebene Bedeutung besitzen, sowie die Derivate der Formel (VI)

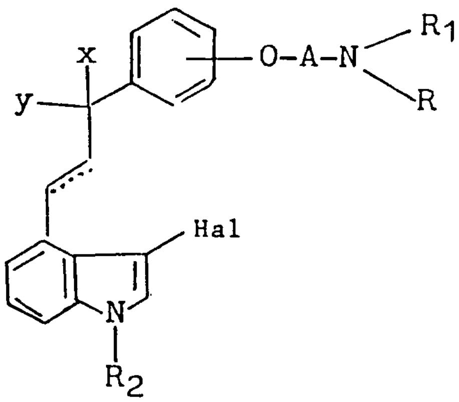

VI

worin R, $R_1$, $R_2$, x, y, A, Hal und die gestrichelte Linie die angegebene Bedeutung besitzen.

**Patentansprüche für folgenden Vertragsstaat : AT**

1. Verfahren zur Herstellung von Hydroxyalkoxy-4-phenylpropylindol-Derivaten und deren Additionssalzen mit Mineral- oder organischen Säuren der allgemeinen Formel (I)

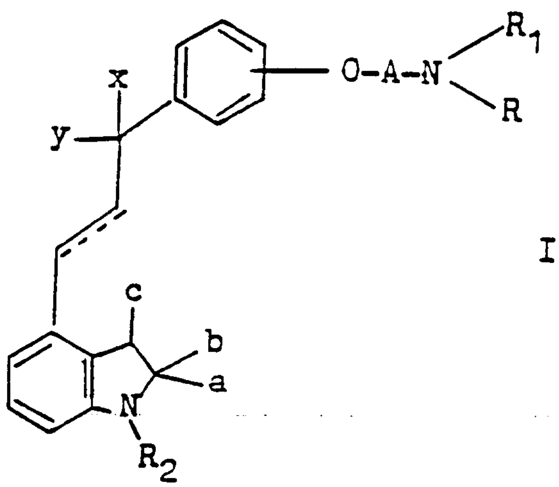

I

worin R und $R_1$, unabhängig voneinander, ein Wasserstoffatom, einen linearen Alkylrest mit 1 bis 5 Kohlenstoffatomen, einen verzweigten Alkylrest mit 3 bis 5 Kohlenstoffatomen bedeuten, a zusammen mit b eine Oxofunktion wiedergibt oder gemeinsam mit c eine Kohlenstoff-Kohlenstoff-Bindung darstellt, b ein Wasserstoffatom oder gemeinsam mit a eine Oxofunktion bedeutet, c ein Wasserstoffatom oder gemeinsam mit a eine Kohlenstoff-Kohlenstoff-Bindung bedeutet, die gestrichelte Linie die etwaige Anwesenheit einer Kohlenstoff-Kohlenstoff-Bindung wiedergibt, A eine Kette

$$-(CH_2)_m-\underset{OH}{\underset{|}{CH}}-CH_2-$$

wiedergibt, worin m die Werte 1, 2 oder 3 annehmen kann, $R_2$ für ein Wasserstoffatom, einen linearen Alkylrest mit 1 bis 5 Kohlenstoffatomen oder einen verzweigten Alkylrest mit 3 bis 5 Kohlenstoffatomen steht, x eine Hydroxygruppe oder ein Wasserstoffatom oder gemeinsam mit y eine Oxofunktion bedeutet, und y ein Wasserstoffatom oder gemeinsam mit x eine Oxofunktion bedeutet, dadurch gekennzeichnet, daß man ein Derivat der Formel (II)

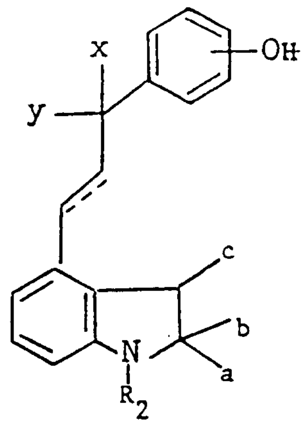

(II)

worin $R_2$, a, b, c, x, y und die gestrichelte Linie die angegebene Bedeutung besitzen, mit einem Halogenid der Formel (III)

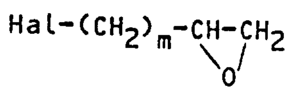

(III)

worin m die angegebene Bedeutung besitzt, und Hal für ein Chlor-, Brom- oder Jodatom steht, umsetzt, um zu einem Produkt der Formel (IV)

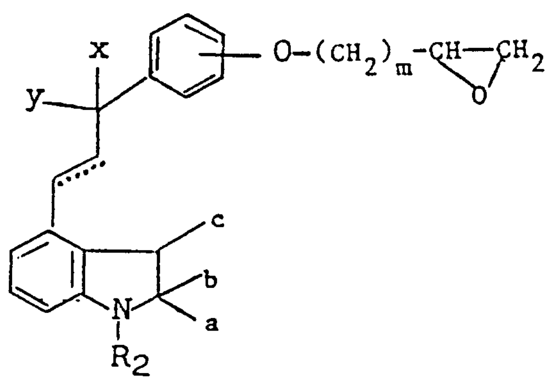

(IV)

worin $R_2$, a, b, c, x, y, m und die gestrichelte Linie die angegebene Bedeutung besitzen, zu gelangen, welches man mit einem Amin der Formel (V)

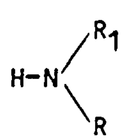

(V)

worin R und $R_1$ die angegebene Bedeutung besitzen, umsetzt, um zu einem Produkt der Formel (I) zu gelangen, welches man isoliert und gewünschtenfalls in ein Salz überführt.

**2.** Verfahren gemäß Anspruch 1, dadurch gekennzeichnet, daß Hal ein Chloratom bedeutet, und daß die Umsetzung des Derivats der Formel (II) mit dem Halogenid der Formel (III) in Anwesenheit einer Base durchgeführt wird.

**3.** Verfahren gemäß Anspruch 1, dadurch gekennzeichnet, daß man ein Produkt der Formel ($II_A$)

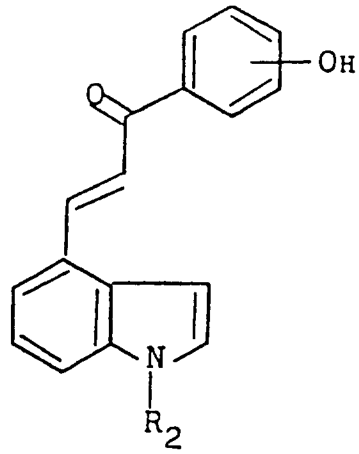

($II_A$)

worin $R_2$ die angegebene Bedeutung besitzt, mit einem Halogenid der Formel (III)

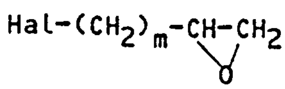

(III)

worin Hal und m die angegebene Bedeutung besitzen, umsetzt, um zu einem Produkt der Formel ($IV_A$)

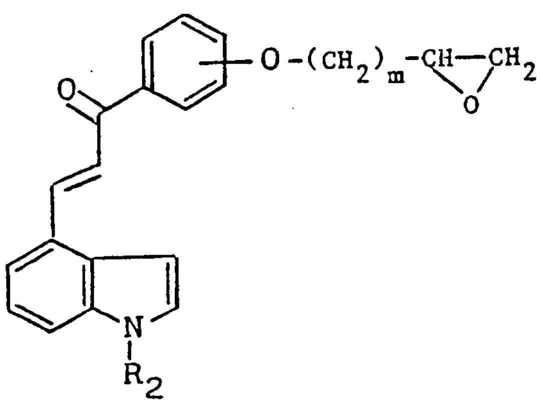

($IV_A$)

worin $R_2$ und m die angegebene Bedeutung besitzen, zu gelangen, welches man mit einem Amin der Formel (V)

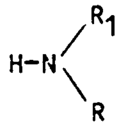

(V)

worin R und $R_1$ die angegebene Bedeutung besitzen, umsetzt, um zu einem Produkt der Formel ($I_A$)

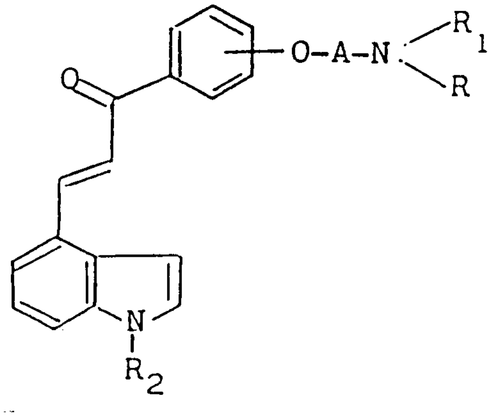

$I_A$

worin R, $R_1$ und $R_2$ die angegebene Bedeutung besitzen, zu gelangen, welches man entweder im Hinblick auf die Doppelbindung der aliphatischen Kette reduziert, um zu einem Produkt der Formel ($I_B$)

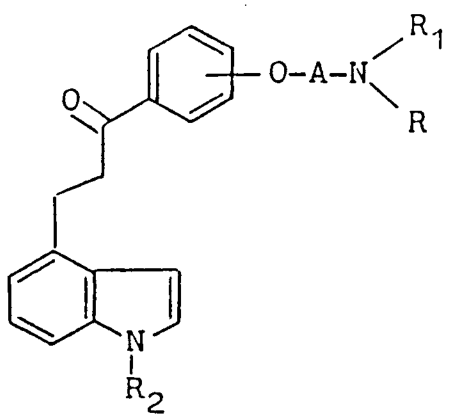

$I_B$

worin A, R, $R_1$ und $R_2$ die angegebene Bedeutung besitzen, zu gelangen, welches man entweder isoliert und gewünschtenfalls in ein Salz überführt oder im Hinblick auf die Ketonfunktion reduziert, um zu einem Produkt der Formel ($I_C$)

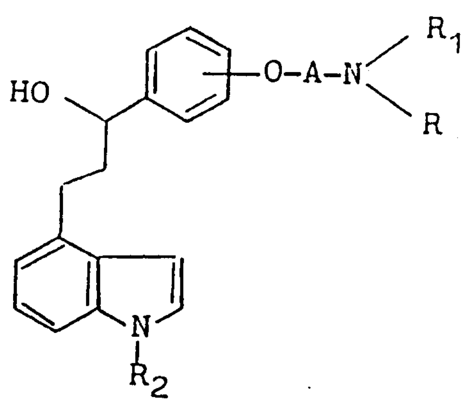

$I_C$

worin A, R, $R_1$ und $R_2$ die angegebene Bedeutung besitzen, zu gelangen, welches man entweder isoliert und gewünschtenfalls in ein Salz überführt, oder im Hinblick auf die Hydroxyfunktion reduziert, um zu einem Produkt der Formel ($I_D$)

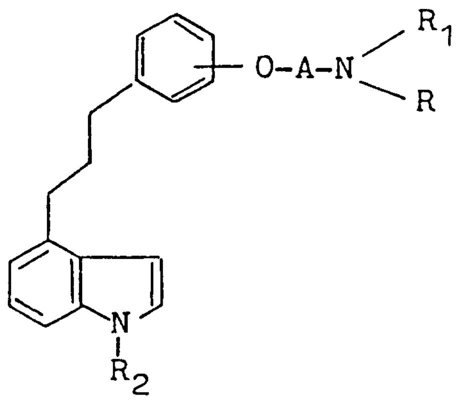

$I_D$

worin A, R, $R_1$ und $R_2$ die angegebene Bedeutung besitzen, zu gelangen, welches man isoliert und gewünschtenfalls in ein Salz überführt, oder das Produkt der Formel ($I_A$) im Hinblick auf die Ketonfunktion reduziert, um zu einem Produkt der Formel ($I_E$)

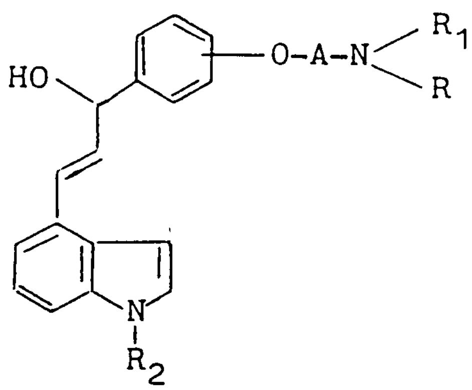

$I_E$

worin A, R, $R_1$ und $R_2$ die angegebene Bedeutung besitzen, zu gelangen, welches man entweder isoliert oder im Hinblick auf die Doppelbindung der aliphatischen Kette reduziert, um zu einem Produkt der Formel ($I_D$) zu gelangen, welches man isoliert und gewünschtenfalls in ein Salz überführt,
oder das Produkt der Formel ($I_A$) gleichzeitig im Hinblick auf die Ketonfunktion und auf die Doppelbindung reduziert, um zu einem Produkt der Formel ($I_D$) zu gelangen, welches man isoliert und gewünschtenfalls in ein Salz überführt,
danach gewünschtenfalls die Produkte der Formel ($I_A$), ($I_B$), ($I_C$), ($I_D$) und ($I_E$) der Einwirkung eines Halogenierungsmittels unterzieht, um zu einem Produkt der Formel (VI)

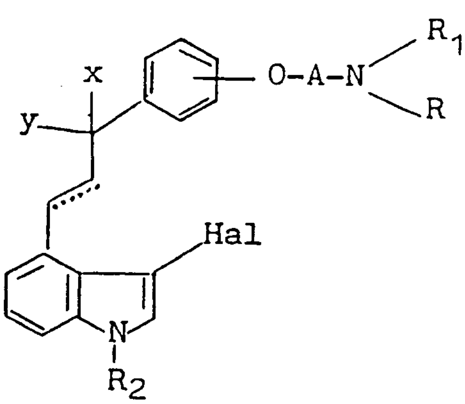

VI

worin Hal für ein Brom- oder Chloratom steht, und x, y, A, R, $R_1$, $R_2$ und die gestrichelte Linie die

angegebene Bedeutung besitzen, zu gelangen, welches man einer Hydrolyse unterzieht, um zu einem Produkt der Formel ($I_F$)

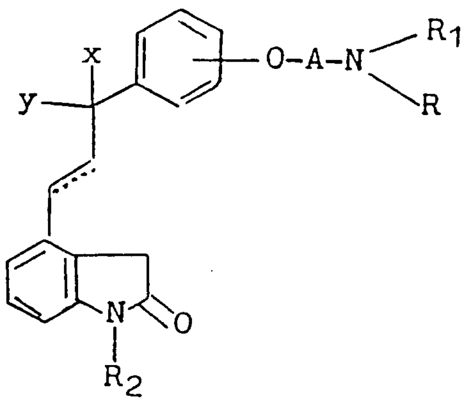

$I_F$

worin x, y, A, R, $R_1$, $R_2$ und die gestrichelte Linie die angegebene Bedeutung besitzen, zu gelangen, welches man isoliert und gewünschtenfalls in ein Salz überführt.

**4.** Verfahren gemäß Anspruch 3, dadurch gekennzeichnet, daß

- die Reduktion der Doppelbindung des Produkts der Formel ($I_A$) mit gasförmigem Wasserstoff in Gegenwart eines Katalysators auf Basis von Platin oder Palladium in einem Lösungsmittel wie einem Alkanol mit 1 bis 5 Kohlenstoffatomen oder durch gasförmigen Wasserstoff in Anwesenheit von Raney-Nickel in einem Lösungsmittel wie Ethylacetat oder durch Einwirken von Natrium in Ammoniak in einem Lösungsmittel wie Tetrahydrofuran durchgeführt wird, wobei die Kontaktdauer weniger als 3 Stunden und vorzugsweise etwa 1 Stunde beträgt;
- die Reduktion der Ketonfunktion des Produkts der Formel ($I_B$) mit einem Alkaliborhydrid oder einem Alkalicyanoborhydrid durchgeführt wird;
- die Reduktion der Hydroxyfunktion des Produkts der Formel ($I_C$) vorzugsweise durch Einwirken von Natrium in Ammoniak durchgeführt wird;
- die Reduktion der Ketonfunktion des Produkts der Formel ($I_A$) durch den zwischen einem Alkaliborhydrid, vorzugsweise Natriumborhydrid, und Pyrridin gebildeten Komplex durchgeführt wird;
- die Reduktion der Doppelbindung des Produkts der Formel ($I_E$) vorzugsweise mit Natrium in Ammoniak durchgeführt wird;
- die gleichzeitige Reduktion der Ketonfunktion und der Doppelbindung des Produkts der Formel ($I_A$) vorzugsweise mit Natrium in Ammoniak durchgeführt wird;
- Hal für ein Chloratom steht.

**5.** Verfahren gemäß einem der Ansprüche 1 bis 4, dadurch gekennzeichnet, daß in dem Produkt der Formel (II) oder ($II_A$) $R_2$ für ein Wasserstoffatom steht, und daß in dem Produkt der Formel (V) $R_1$ ein Wasserstoffatom bedeutet.

**6.** Verfahren gemäß einem der Ansprüche 1 bis 4, dadurch gekennzeichnet, daß in dem Produkt der Formel (II) oder ($II_A$) $R_2$ für ein Wasserstoffatom steht, und daß in dem Produkt der Formel (V) $R_1$ für einen Propylrest steht.

**7.** Verfahren gemäß Anspruch 5, dadurch gekennzeichnet, daß man das 1-[2-[3-[(1,1-Dimethylethyl)-amino]-2-hydroxypropoxy]-phenyl]-3-(1H-indol-4-yl)-propanon sowie dessen Additionssalze mit Mineral- oder organischen Säuren herstellt.

**8.** Verfahren gemäß Anspruch 6, dadurch gekennzeichnet, daß man das 1,3-Dihydro-4-[3-[2-[3-propylamino]-2-hydroxypropoxy]-phenyl]-propyl]-2H-indol-2-on sowie dessen Additionssalze mit Mineral- oder organischen Säuren herstellt.